# DEMANDE DE BREVET EUROPEEN

(11) **EP 4 428 253 A1**
(43) Date de publication de la demande: **11.09.2024**
(21) Numéro de dépôt: 23161246.6
(22) Date de dépôt: 10.03.2023
(51) Int. Cl.: C12Q 1/689

(54) **METHODE DE DETERMINATION DE LA SUSCEPTIBILITE D'UNE SOUCHE DE L'ESPECE MYCOBACTERIUM TUBERCULOSIS AU PYRAZINAMIDE**

(71) Demandeur: BIOMÉRIEUX, 69280 Marcy L'Etoile (FR)
(72) Inventeur: MAHE, Pierre, 38250 LANS EN VERCORS (FR); TOURNOUD, Maud, 38360 SASSENAGE (FR); BARLAS, Philippine, 38500 LA BUISSE (FR); JAILLARD DANCETTE, Magali, 69200 LYON (FR)
(74) Mandataire: bioMérieux PI Groupement mandataires

(57) **Abrégé**

L'invention une méthode de détermination de la susceptibilité d'une souche de l'espèce *Mycobacterium tuberculosis* au pyrazinamide, ladite méthode comprenant les étapes suivantes de (i) détermination de la présence dans le génome de ladite souche d'au moins une mutation dans le gène *rpoB* entre les positions 761112 et 761182, et (ii) détermination du caractère sensible ou résistant de ladite souche en fonction de la présence ou non de ladite au moins une mutation. L'invention concerne également un kit comprenant des moyens de détection d'au moins une séquence choisie parmi les séquences SEQ ID NO :1 à 3, et l'utilisation de ce même kit pour la détermination de la susceptibilité d'une souche de l'espèce *Mycobacterium tuberculosis* au pyrazinamide.

## Description

### DOMAINE DE L'INVENTION

La présente invention a trait au domaine technique de la biologie moléculaire appliquée à la génomique bactérienne, et en particulier au domaine de la détermination de la résistance aux antibiotiques à partir de l'analyse de leur génome. L'invention concerne particulièrement une méthode pour déterminer la résistance d'une souche de l'espèce *Mycobacterium tuberculosis* à un antibiotique particulier, à savoir au pyrazinamide.

### ARRIERE-PLAN TECHNOLOGIQUE

La tuberculose (TB) est une maladie infectieuse des voies respiratoires humaines qui touche près d'un tiers de la population mondiale et représente un problème mondial de santé.

*Mycobacterium tuberculosis* est la bactérie responsable de la tuberculose et bien que des vaccins soient disponibles, leur effet diminue avec le temps et les patients infectés sont généralement traités avec des antibiotiques comme l'isoniazide, la rifampicine ou encore la pyrazinamide pour contrôler la maladie.

Appartenant au genre des mycobactéries *(Mycobacterium spp.)* au même titre que le bacille de la lèpre *(Mycobacterium leprae* ou bacille de Hansen), ou les mycobactéries dites atypiques, elle est découverte par Robert Koch en 1882 et son génome est séquencé en 1998.

Au cours des dernières années, le développement de souches *Mycobacterium tuberculosis* multi-résistantes, ultrarésistantes et totalement résistantes aux médicaments tels que les antibiotiques, a été signalé.

La multi-résistance aux antibiotiques implique la résistance de la souche microbienne à tout médicament antituberculeux de première intention, y compris l'isoniazide et/ou la rifampicine, mais peut également impliquer la résistance à tout médicament de deuxième intention, tel que par exemple le pyrazinamide.

Une antibiothérapie inadéquate ou retardée a un effet négatif en ce qu'elle favorise la sélection de mutations spontanées en faveur des souches résistantes par la pression de sélection, aggravant ainsi le problème de la résistance aux antibiotiques. Par conséquent, le développement d'une détection rapide et précise du profil de résistance de *Mycobacterium tuberculosis* présente un intérêt de santé publique mondial.

La susceptibilité d'une souche bactérienne à un antibiotique, c'est-à-dire son caractère sensible ou résistant dans le cadre d'un traitement à base de l'antibiotique administré à un humain ou un animal, n'est pas directement observable par un être humain. En effet, une observation directe de la souche, même au travers de microscopes, ne permet pas de déterminer son comportement face à l'antibiotique.

Le diagnostic *in vitro* en matière bactérienne consiste, par nature, à rendre ce caractère phénotypique observable et donc *in fine* exploitable pour un clinicien. Au cours du XXe siècle les technologies de diagnostic *in vitro* ont essentiellement combiné des techniques de préparation d'échantillon à base de culture, notamment pour rendre visibles et manipulables les souches bactériennes présentes dans les échantillons, et des techniques de mesures optiques du comportement des souches en présence d'un antibiotique.

Par exemple, un flux de travail classique d'un laboratoire microbiologique consiste dans un premier temps à étaler sur un milieu de culture un échantillon prélevé sur un patient suspecté d'infection bactérienne afin de faire apparaître, après incubation, des colonies bactériennes visibles par un opérateur humain ou un automate. Dans un second temps, la taille des colonies étant suffisante, un technicien ou un automate prélève une colonie, la mélange à un antibiotique à différentes concentrations et introduit les mélanges dans un dispositif qui mesure la densité optique de chaque mélange et en déduit la susceptibilité à l'antibiotique. La densité optique signant la prolifération bactérienne, elle caractérise alors sans ambiguïté la sensibilité ou la résistance de la bactérie : si la densité augmente, cela signifie qu'elle prolifère malgré la présence de l'antibiotique, et donc qu'elle est résistante à ce dernier à la concentration antibiotique considérée.

Par exemple, le document US7335485 décrit une méthode de détermination de la susceptibilité d'un microorganisme à un antibiotique, dans laquelle ledit microorganisme est mis en culture en présence de l'antibiotique à tester.

La combinaison des technologies de préparation d'échantillon et des technologies de mesures optiques à base de densité optique montre d'importantes limitations face à une évolution rapide à l'échelle mondiale dans le règne des procaryotes, à savoir l'acquisition de multi-résistances aux antibiotiques, multi-résistances dont on estime qu'elles feront, en 2050, plus de morts que le cancer. En fonction du milieu de culture choisi, certaines souches vont pousser et d'autres non, de sorte que ces technologies ne permettent pas de caractériser la susceptibilité aux antibiotiques de toutes les espèces bactériennes. Ensuite, ces techniques sont extrêmement lentes car elles se basent sur une culture bactérienne qui prend beaucoup de temps. C'est ainsi qu'obtenir un antiobiogramme d'une bactérie prend au moins 30h heures à partir du prélèvement de l'échantillon. Ce délai ne permet pas un traitement efficace des patients qui se voient administrer en première intention, et de manière systématique, un cocktail antibiotique à large spectre. Outre les conséquences pour le patient, cette administration inappropriée et massive d'antibiotique renforce la pression de sélection des bactéries multi-résistantes et contribue donc à leur expansion.

A ce jour donc, on estime que les technologies classiques de diagnostic *in vitro* sont de moins en moins adaptées au traitement des patients et sont, dans une certaine mesure, une des raisons de l'apparition des multi-résistances.

Plus récemment, des technologies sensibles comme la spectrométrie de masse sont appliquées pour déterminer la résistance aux antibiotiques, mais cela nécessite toujours la culture du micro-organisme à tester en présence de l'antibiotique à tester. En outre, dans toutes ces techniques, chaque micro-organisme à tester doit être testé contre des antibiotiques individuels ou des combinaisons d'antibiotiques, ce qui nécessite des tests extensifs, longs et fastidieux.

La maturation des technologies de biologie moléculaire, en particulier les technologies de caractérisations de l'ADN et/ou l'ARN bactérien, telle que la réaction en chaîne par polymérase (ou PCR), les puces à ADN ou le séquençage, réalise un changement de paradigme dans l'analyse de la résistance aux antibiotiques dans les laboratoires.

Tout d'abord, elles sont plus agnostiques vis-à-vis des espèces bactériennes. Par exemple, la technologie métagénomique permet de traiter l'ADN bactérien dans un échantillon biologique quelles que soient les espèces bactériennes en présence. Ensuite, elles ont pour objectif de rendre un résultat en quelques heures, certaines comme la PCR permettant même un résultat en moins de 20 minutes. En contrepartie, les techniques moléculaires de caractérisation de la susceptibilité aux antibiotiques reposent sur des signatures génomiques (absence/présence de gènes, de mutations génétiques, modèles de prédiction, etc.) caractérisant ladite susceptibilité.

De manière non limitative, dans le cadre d'un flux de travail microbiologique pour le traitement d'un patient suspecté d'une infection bactérienne, il existe deux technologies de caractérisation de l'ADN bactérien, à savoir une technologie PCR et une technologie de séquençage de génome complet (ou séquençage WGS pour « whole genome sequencing »). Les deux flux débutent par le prélèvement d'un échantillon biologique sur le patient, suivi par l'application de la technologie PCR ou de la technologie WGS, rendant chacune un résultat de signatures génomiques caractérisant la susceptibilité à un ou plusieurs antibiotiques, résultat sur la base duquel un traitement antibiotique est choisi, puis administré au patient par un clinicien. De manière classiques selon les méthodes connues de la personne du métier, chacune des technologies moléculaires nécessite la préparation de l'échantillon prélevé préalablement à l'application de la PCR elle-même, par exemple une PCR de type « nested » mise en oeuvre par une plateforme FilmArray^{®} de la société BioFire^{®}, ou à l'application du séquençage, par exemple un séquençage de type SBS (Sequencing By Synthesis) mise en oeuvre par une plateforme MiSeq de la société Illumina.

Le document WO2018/065830 décrit par exemple une méthode de PCR quantitative en temps réel (qPCR) pour déterminer le profil de résistance de *Mycobacterium tuberculosis* aux antibiotiques.

Le document CN101580879 décrit une puce génétique et une méthode de détection des mutations génétiques communes de *Mycobacterium tuberculosis* responsables de la résistance aux antibiotiques tels que l'isoniazide, la rifampicine, le streptomycine, l'éthambutol ou encore le pyrazinamide.

Le document IN201941006113 décrit une méthode pourtesterla résistance aux antibiotiques d'une souche *Mycobacterium tuberculosis* à partir d'un échantillon clinique contenant ladite souche, par la mise en oeuvre d'une étape d'identification de variants mononucléotidique (SNVs) à partir des gènes associés à la résistance aux antibiotiques de ladite souche.

D'une manière plus générale, aussi bien chez les bactéries que chez les humains, il est connu que la résistance aux antibiotiques peut être associée au polymorphisme génétique. En se focalisant plus particulièrement sur les signatures génomiques, les premières approches consistaient à identifier dans le génome bactérien des marqueurs de résistance aux antibiotiques identifiés au préalable, approches qualifiées « d'association directe ». Si ces approches sont efficaces lorsque les mécanismes génétiques entrainant la résistance sont bien connus et simples, elles peuvent souffrir d'importantes limitations : connaissance incomplète des mécanismes de résistances chez beaucoup d'espèces et d'antibiotiques, se traduisant par exemple par des bases de données incomplètes, difficulté à tenir compte de différences dans les pouvoirs prédictifs des marqueurs et de l'aspect multifactoriel de la susceptibilité aux antibiotiques (e.g. épistasie, combinaison de multiples mutations,...), etc.

Face à ces difficultés, le déterminisme génétique de la susceptibilité aux antibiotiques est appréhendé plus efficacement par de nouvelles approches à base de technologies informatiques avancées, et en particulier par des technologies d'apprentissage automatique supervisé dont l'architecture d'apprentissage et d'application peut être résumé de la manière suivante :
A. pour un ensemble de souches bactériennes d'apprentissage :
   - A.1 chaque souche est séquencée et phénotypiquement caractérisée (e.g. mesure de sa concentration minimale inhibitrice et/ou mesure de sa susceptibilité - résistance, intermédiaire ou sensible- pour un ou plusieurs antibiotiques).
   - A.2. un modèle informatique de prédiction de la susceptibilité à l'antibiotique est appris en fonction des génomes et des données phénotypiques.
B. pour une nouvelle souche dont on cherche à connaître la susceptibilité à un antibiotique de l'étape (A.1),
B.1. la souche est séquencée ;
B.2. la modèle de prédiction informatique est appliqué à son génome numérique afin de déterminer sa susceptibilité.

De tels modèles d'apprentissage et de prédictions sont par exemple décrits dans les documents WO2021180768 et WO2021180771 dont la Demanderesse est titulaire.

Ainsi, il existe un besoin de développer de nouvelles signatures génomiques afin de renforcer l'arsenal disponible pour l'identification de la susceptibilité aux antibiotiques, et notamment au pyrazinamide. En effet, les signatures sont obtenues par des modèles d'apprentissage dont la limite réside dans la taille et la diversité des bases génomiques utilisées pour entrainer le modèle. Par conséquent, il peut être avantageux de combiner l'utilisation simultanée de plusieurs signatures afin de déterminer avec encore plus de précision la susceptibilité à un antibiotique. Dans ce contexte, il est pertinent d'identifier de nouvelles signatures génomiques présentant un niveau de performance suffisant, à savoir notamment une spécificité d'au moins 90%, pour déterminer la susceptibilité d'une souche de l'espèce *Mycobacterium tuberculosis* au pyrazinamide.

### RESUME DE L'INVENTION

Après de nombreuses recherches sur le génome de l'espèce *Mycobacterium tuberculosis,* il est du mérite des inventeurs d'avoir identifié une nouvelle signature génomique permettant de déterminer la susceptibilité d'une souche de ladite espèce au pyrazinamide. En d'autres termes, la détermination de la susceptibilité selon la présente invention permet d'identifier si une souche de l'espèce *Mycobacterium tuberculosis* est sensible ou résistante au pyrazinamide, et ce, avec une spécificité de plus de 90%. Ainsi, la présente invention permet de lutter contre la résistance aux antibiotiques en évitant notamment qu'un traitement au pyrazinamide soit initié alors que la souche est résistante.

De plus, il est connu que la présence de mutations dans le gène *pncA* est corrélée à la résistance des souches l'espèce *Mycobacterium tuberculosis* au pyrazinamide. A l'inverse, et de manière tout à fait surprenante, la détermination du caractère sensible ou résistant d'une souche *Mycobacterium tuberculosis* au pyrazinamide est réalisée à partir de l'identification de la présence ou absence de mutations dans des gènes qui ne sont pas corrélés de manière constitutive à la résistance audit antibiotique, comme le gène *rpoB* ou encore les gènes *gyrA, embB* ou *katG.*

Un premier objet de l'invention concerne ainsi une méthode pour déterminer la susceptibilité d'une souche de l'espèce *Mycobacterium tuberculosis* au pyrazinamide, ladite méthode comprenant les étapes suivantes de détermination de la présence dans le génome de ladite souche d'au moins une mutation dans le gène *rpoB* entre les positions 761112 et 761182, et de détermination du caractère sensible ou résistant de ladite souche sur la base de(s) la mutation(s) identifiée(s).

De préférence, la méthode comprend également les étapes suivantes de détermination de la présence ou absence d'au moins une mutation dans le gène *fabG1* entre les positions 1673413 et 1673454, éventuellement aussi dans le gène *gyrA* entre les positions 7552 et 7582, éventuellement aussi dans le gène *Rv1042c* et sa région promotrice entre les positions 1165444 et 1165528, éventuellement aussi dans le gène *Rv1149* et sa région promotrice entre les positions 1277873 et 1277957, éventuellement aussi dans le gène *embB* entre les positions 4247581 et 4247622, éventuellement aussi dans le gène *pncA* entre les positions 2288853 et 2289239, et enfin, éventuellement aussi dans le gène *katG* entre les positions 2155164 et 2155205.

Un autre objet de l'invention concerne un kit comprenant des moyens de détection et/ou d'amplification d'au moins une séquence choisie parmi les séquences SEQ ID NOs :1 à 3, et éventuellement au moins une autre séquence choisie parmi les séquences SEQ ID NOs : 4 à 12, dans le génome d'une souche de l'espèce *Mycobacterium tuberculosis,* de préférence dans le gène *rpoB* de ladite souche, ainsi que l'utilisation dudit kit pour la détermination de la susceptibilité de la souche au pyrazinamide.

La méthode selon l'invention pouvant être mise en oeuvre par ordinateur, un autre objet concerne enfin un appareil de traitement de donnée comprenant :
(a) des moyens pour mettre en oeuvre la méthode selon l'invention, notamment des moyens pour déterminer la présence de mutations dans le gène *rpoB* de la souche *Mycobacterium tuberculosis* entre les positions 761112 et 761182, et/ou des moyens permettant de comparer le séquence dudit gène avec le génome de référence *Mycobacterium tuberculosis* H37Rv (référence NC_000962.3), et/ou des moyens pour identifier la présence des séquences SEQ ID NOs 1 à 3 dans le génome de la souche *Mycobacterium tuberculosis,* et/ou des moyens pour fournir une donnée de sortie de la présence ou de l'absence desdites séquences, lorsque lesdits moyens sont mis en oeuvre ou pilotés par ordinateur, ou
(b) un processeur adapté à ou configuré pour exécuter la méthode mise en oeuvre par ordinateur selon la revendication 16, en particulier un processeur adapté à ou configuré pour exécuter les étapes de ladite méthode.

### DESCRIPTION DETAILLEE DE L'INVENTION

Dérivé de la nicotinamide, le pyrazinamide est un médicament antituberculeux permettant d'obtenir *in vivo,* aux posologies indiquées, une action bactéricide sur les bacilles tuberculeux intracellulaires (se trouvant donc dans un milieu acide, condition nécessaire à l'action du pyrazinamide). Le pyrazinamide est donc utilisé dans la prise en charge de tuberculoses. L'espèce *Mycobacterium bovis* et les mycobactéries atypiques lui sont naturellement résistantes. A l'inverse, l'espèce *Mycobacterium tuberculosis* et une espèce très proche, le *Mycobacterium africanum,* lui sont régulièrement sensibles.

Comme mentionné précédemment, une antibiothérapie inadéquate ou retardée a un effet négatif favorisant la sélection de mutations spontanées en faveur des souches résistantes par la pression de sélection, aggravant ainsi le problème de la résistance aux antibiotiques.

Par conséquent, lors de la prise en charge d'un patient tuberculeux, il est primordial de pouvoir s'assurer que la souche *Mycobacterium tuberculosis* doit-il est infecté, ne présente pas un caractère résistant au pyrazinamide.

Ainsi, un premier objet de l'invention concerne une méthode de détermination de la susceptibilité d'une souche de l'espèce *Mycobacterium tuberculosis* au pyrazinamide, ladite méthode comprenant une étape de détermination de la présence dans le génome de ladite souche d'au moins une mutation dans le gène *rpoB* entre les positions 761112 et 761182, et une étape de détermination du caractère sensible ou résistant de ladite souche en fonction de la présence ou non de mutations dans ledit gène.

De manière tout à fait surprenante, comme expliqué précédemment, la présente méthode permet de déterminer la susceptibilité au pyrazinamide à partir de l'identification de mutations dans des gènes qui ne sont pas associés de manière constitutive à la résistance à pyrazinamide mais à d'autres antibiotiques. En effet, l'identification de mutations dans le gène *rpoB* est normalement associé à la résistance à la rifamycine et ses dérivés tels que la rifampicine.

Sauf précision explicite, les termes techniques et scientifiques utilisés dans la présente description ont la même signification que celle couramment comprise par la personne du métier du domaine de la présente description.

L'expression « détermination de la susceptibilité » fait référence à la détermination du caractère sensible ou résistant de la souche au pyrazinamide.

Pour la suite de la description, le terme « souche(s) » fait référence aux souches de l'espèce *Mycobacterium tuberculosis,* à moins que le contexte permette clairement d'identifier qu'il s'agisse d'une autre espèce.

### Détermination de la présence d'au moins une mutation dans le gène rpoB

Le terme "mutation" se rapporte à une variation de la séquence par rapport à une séquence de référence. Une telle séquence de référence peut être une séquence déterminée dans un organisme de type sauvage prédominant ou un organisme de référence telle qu'une souche bactérienne définie et connue par exemple. Une mutation est par exemple une délétion d'un ou plusieurs nucléotides, une insertion d'un ou plusieurs nucléotides, ou une substitution d'un ou plusieurs nucléotides, une duplication d'un ou une séquence de plusieurs nucléotides, une translocation d'un ou une séquence de plusieurs nucléotides, et, en particulier, un polymorphisme mononucléotidique (SNP).

Le génome de *Mycobacterium tuberculosis* a été entièrement séquencé en 1998. Cette espèce possède un chromosome circulaire de 4 411 529 paires de bases (GC%=65.6) pour 3924 gènes.

Dans le cadre de la présente invention, la détermination de la présence des mutations dans un ou plusieurs gène de la souche est réalisée par rapport au génome sauvage de référence de *Mycobacterium tuberculosis,* à savoir *Mycobacterium tuberculosis* H37Rv. Ce génome sauvage de référence est notamment indexé dans la base NCBI sous la référence NC_000962.3. Ainsi, toutes les positions génomiques selon la présente description sont données par rapport audit génome NC_000962.3.

La comparaison peut être effectuée selon les méthodes connues de la personne du métier pour comparer deux génomes entre eux, ou plus précisément, des positions génomiques particulières entre deux génomes, à savoir notamment par bio-informatique.

Ainsi, selon la présente invention, la détermination de la présence dans le génome de la souche d'au moins une mutation dans le gène *rpoB* entre les positions 761112 et 761182 par rapport au génome sauvage *Mycobacterium tuberculosis* H37Rv permet d'identifier le caractère sensible ou résistant de ladite souche à la pyrazinamide.

Au sens de la présente invention, l'identification ou la détermination du caractère sensible ou résistant d'une souche *Mycobacterium tuberculosis* s'entend comme la prédiction, avec un certain degré d'erreur associé, dudit caractère.

La détermination de la présence d'au moins une mutation dans un ou plusieurs gènes d'une souche de *Mycobacterium tuberculosis* peut être réalisée selon les méthodes connues par de la personne du métier. A titre d'exemple, il est possible de mettre en oeuvre une méthode d'hybridation, d'amplification ou de séquençage.

Selon un mode de réalisation particulier, la détermination de la présence de mutations est réalisée par une technique d'hybridation, de préférence avec des micropuces d'hybridation ou par des techniques du type NanoString^{®} nCounter^{®}, par une technique d'amplification, de préférence par PCR ou qPCR, ou par une technique de séquençage, de préférence par un séquençage haut débit ou un séquençage par synthèse.

Selon un mode de réalisation préféré, la méthode comprend avant l'étape de détermination de la présence de mutations dans les gènes de la souche *Mycobacterium tuberculosis,* une étape préalable de caractérisation de l'ADN de ladite souche. Cette étape est réalisée selon les méthodes connues de la personne du métier, de préférence par PCR ou séquençage (par exemple WGS).

La méthode peut également comprendre une étape d'obtention préalable d'un échantillon biologique issu d'un sujet, ledit échantillon étant susceptible de contenir au moins une souche de l'espèce *Mycobacterium tuberculosis.*

Selon un mode de réalisation particulier, l'étape de détermination de la présence d'au moins une mutation dans le gène *rpoB* comprend la détermination de la présence d'au moins une mutation entre les positions 761151 et 761182, et/ou entre les positions 761112 et 761142, et/ou entre les positions 761138 et 761173.

Selon une variante préférée de ce mode de réalisation, l'étape de détermination de la présence d'au moins une mutation dans le gène *rpoB* de la souche comprend la détermination dans ce gène de la présence d'au moins une séquence choisie parmi les séquences SEQ ID NO :1, SEQ ID NO :2 et SEQ ID NO :3, de préférence la séquence SEQ ID NO : 2, et de préférence encore, les séquences SEQ ID NOs 1 à 3. D'après la méthode de l'invention, le caractère sensible ou résistant de la souche est déterminé en fonction de la présence ou non de mutation(s) entre les positions décrites précédemment, et en particulier, en fonction de la présence ou non d'une ou plusieurs séquences choisies parmi les séquences SEQ ID NOs :1 à 3. Le tableau 1 dans les exemples de réalisation reprend les différentes conclusions quant au caractère sensible ou résistant de ladite souche en fonction de la présence ou non desdites séquences.

### Détermination de la présence d'au moins une mutation dans un ou plusieurs gènes additionnels

Les performances de la méthode selon la présente invention peuvent être améliorées en combinant la détermination de la présence de mutation(s) dans un ou plusieurs gènes additionnels de la souche de *Mycobacterium tuberculosis.*

Ainsi, selon un mode de réalisation particulier, la méthode comprend en outre une étape de détermination de la présence dans le génome de la souche d'au moins une mutation dans le gène *fabG1* entre les positions 1673413 et 1673454, et de préférence, entre les positions 1673415 et 1673454 et/ou entre les positions 1673413 et 1673444.

Selon une variante préférée de ce mode de réalisation, la détermination de la présence d'au moins une mutation dans le gène *fabG1* entre lesdites positions génomiques comprend avantageusement la détermination de la présence de la séquence SEQ ID NO :4 et/ou SEQ ID NO :5, de préférence les deux.

De manière avantageuse, la méthode selon l'invention comprend ainsi une étape de détermination dans le génome d'une souche *Mycobacterium tuberculosis* de la présence d'au moins une mutation dans les gènes *rpoB* et *fabG1,* ladite étape comprenant la détermination de la présence des séquences SEQ ID NO :1, SEQ ID NO :2 et SEQ ID NO :3 dans le gène *rpoB,* et des séquences SEQ ID NO :4 et SEQ ID NO :5 dans le gène *fabG1,* ainsi qu'une étape de détermination du caractère sensible ou résistant en fonction de la présence ou non desdites séquences. Le tableau 2 dans les exemples de réalisation reprend les différentes conclusions quant au caractère sensible ou résistant de ladite souche en fonction de la présence ou non desdites séquences.

Selon un autre mode de réalisation particulier, la méthode selon la présente invention comprend en outre une étape de détermination de la présence dans le génome de la souche d'au moins une mutation dans le gène *gyrA* entre les positions 7552 et 7582. Selon une variante préférée de ce mode de réalisation, la détermination de la présence d'au moins une mutation dans le gène *gyrA* entre lesdites positions génomiques comprend avantageusement la détermination de la présence de la séquence SEQ ID NO :6.

Ainsi, de manière tout à fait avantageuse, la méthode selon l'invention comprend une étape de détermination dans le génome d'une souche *Mycobacterium tuberculosis* de la présence d'au moins une mutation dans les gènes *rpoB, fabG1* et *gyrA,* ladite étape comprenant la détermination de la présence des séquences SEQ ID NOs :1 à 6 dans lesdits gènes respectifs, et une étape de détermination du caractère sensible ou résistant en fonction de présence ou non desdites séquences. Le tableau 3 dans les exemples de réalisation reprend les différentes conclusions quant au caractère sensible ou résistant de ladite souche en fonction de la présence ou non desdites séquences.

Selon un autre mode de réalisation, la méthode selon la présente invention comprend en outre une étape de détermination de la présence dans le génome de la souche d'au moins une mutation dans le gène *Rv1042c* et sa région promotrice entre les positions 1165444 et 1165528, et/ou d'au moins une mutation dans le gène *Rv1149* et sa région promotrice entre les positions 1277873 et 1277957.

De préférence selon ce mode de réalisation, la méthode comprend une étape de détermination de la présence d'au moins une mutation dans le gène *Rv1042c* et sa région promotrice entre les positions 1165444 et 1165492 et/ou entre les positions 1165497 et 1165528, et de détermination d'au moins une mutation dans le gène *Rv1149* et sa région promotrice entre les positions 1277873 et 1277904 et/ou entre les positions 1277909 et 1277957.

Selon une première variante de ce mode de réalisation, la méthode comprend une étape de détermination de la présence d'au moins une mutation dans le gène *Rv1042c* entre les positions 1165444 et 1165492 et d'au moins une mutation dans le gène *Rv1149* entre les positions 1277909 et 1277957. Selon cette variante, la détermination de la présence d'au moins une mutation dans ledit gène comprend avantageusement la détermination de la présence de la séquence SEQ ID NO :7

Selon une deuxième variante de ce mode de réalisation, la méthode comprend une étape de détermination de la présence d'au moins une mutation au niveau du gène Rv1042c et de sa région promotrice entre les positions 1165497 et 1165528 et d'au moins une mutation au niveau du gène *Rv1149* et de sa région promotrice entre les positions 1277873 et 1277904. Selon cette variante, la détermination de la présence d'au moins une mutation dans ledit gène comprend avantageusement la détermination de la présence de la séquence SEQ ID NO :8.

Selon une troisième variante préférée de ce mode de réalisation, la détermination de la présence d'au moins une mutation dans le gène *Rv1042c* et/ou *Rv1149,* et leurs régions promotrices respectives, entre lesdites positions génomiques comprend avantageusement la détermination de la présence de la séquence SEQ ID NO :7 et/ou de la séquence SEQ ID NO :8, de préférence les deux.

Ainsi, selon un autre mode de réalisation préféré, la méthode selon l'invention comprend une étape de détermination dans le génome d'une souche *Mycobacterium tuberculosis* de la présence d'au moins une mutation dans les gènes *rpoB, fabG1, gyrA, Rv1042c* et *Rv1149*, ladite étape comprenant la détermination de la présence des séquences SEQ ID NO :1 à SEQ ID NO :8 dans lesdits gènes respectifs, et une étape de détermination du caractère sensible ou résistant en fonction de présence ou non desdites séquences. Le tableau 4 dans les exemples de réalisation reprend les différentes conclusions quant au caractère sensible ou résistant de ladite souche en fonction de la présence ou non desdites séquences.

Selon un autre mode de réalisation, la méthode selon la présente invention comprend en outre une étape de détermination de la présence dans le génome de la souche d'au moins une mutation dans le gène *embB* entre les positions 4247581 et 4247622. De préférence, selon ce mode de réalisation, la détermination de la présence d'au moins une mutation dans le gène *embB* entre lesdites positions génomiques comprend avantageusement la détermination de la présence de la séquence SEQ ID NO :9.

Ainsi, selon un autre mode de réalisation préféré, la méthode selon l'invention comprend une étape de détermination dans le génome d'une souche *Mycobacterium tuberculosis* de la présence d'au moins une mutation dans les gènes *rpoB, fabG1, gyrA, Rv1042c, Rv1149* et *embB,* ladite étape comprenant la détermination de la présence des séquences SEQ ID NO :1 à SEQ ID NO :9 dans lesdits gènes respectifs, et une étape de détermination du caractère sensible ou résistant en fonction de présence ou non desdites séquences. Le tableau 5 dans les exemples de réalisation reprend les différentes conclusions quant au caractère sensible ou résistant de ladite souche en fonction de la présence ou non desdites séquences.

Selon un autre mode de réalisation, la méthode selon la présente invention comprend en outre une étape de détermination de la présence dans le génome de la souche d'au moins une mutation dans le gène *pncA* entre les positions 2288853 et 2289239, et de préférence entre les positions 2288853 et 2288885 et/ou entre les positions 2289209 et 2289239.

Selon une variante préférée de ce mode de réalisation, la détermination de la présence d'au moins une mutation dans le gène *pncA* entre lesdites positions génomiques comprend avantageusement la détermination de la présence de la séquence SEQ ID NO :10 et/ou de la séquence SEQ ID NO :11.

Ainsi, selon un autre mode de réalisation préféré, la méthode selon l'invention comprend une étape de détermination dans le génome d'une souche *Mycobacterium tuberculosis* de la présence d'au moins une mutation dans les gènes *rpoB, fabG1, gyrA, Rv1042c, Rv1149*, *embB* et *pncA,* ladite étape comprenant la détermination de la présence des séquences SEQ ID NO :1 à SEQ ID NO :11 dans lesdits gènes respectifs, et une étape de détermination du caractère sensible ou résistant en fonction de présence ou non desdites séquences. Le tableau 6 dans les exemples de réalisation reprend les différentes conclusions quant au caractère sensible ou résistant de ladite souche en fonction de la présence ou non desdites séquences.

Selon un autre mode de réalisation, la méthode selon la présente invention comprend en outre une étape de détermination de la présence dans le génome de la souche d'au moins une mutation dans le gène *katG* entre les positions 2155164 et 2155205.

Selon une variante préférée de ce mode de réalisation, la détermination de la présence d'au moins une mutation dans le gène *katG* entre lesdites positions génomiques comprend avantageusement la détermination de la présence de la séquence SEQ ID NO :12.

Selon un autre mode de réalisation préféré, la méthode de détermination de la susceptibilité de la souche au pyrazinamide d'une souche de l'espèce *Mycobacterium tuberculosis* comprend les étapes suivantes de :
- détermination de la présence ou absence d'au moins une mutation dans le gène *rpoB* entre les positions 761112 et 761182,
- détermination de la présence ou absence d'au moins une mutation dans le gène *fabG1* entre les positions 1673413 et 1673454,
- détermination de la présence ou absence d'au moins une mutation dans le gène *gyrA* entre les positions 7552 et 7582,
- détermination de la présence ou absence d'au moins une mutation dans le gène *Rv1042c* entre les positions 1165444 et 1165492 et/ou dans la région génomique entre les positions 1165497 et 1165528, et d'au moins une mutation dans le gène *Rv1149* entre les positions 1277909 et 1277957, et/ou dans la région génomique entre les positions 1277873 et 1277904,
- détermination de la présence ou absence d'au moins une mutation dans le gène *embB* entre les positions 4247581 et 4247622,
- détermination de la présence ou absence d'au moins une mutation dans le gène *pncA* entre les positions 2288853 et 2289239,
- détermination de la présence ou absence d'au moins une mutation dans le gène *katG* entre les positions 2155164 et 2155205, et
- détermination du caractère sensible ou résistant de ladite souche en fonction de la présence ou non de desdites mutations.

Selon un autre mode de réalisation préféré, la méthode selon l'invention comprend une étape (a) de détermination dans le génome d'une souche *Mycobacterium tuberculosis* de la présence d'au moins une mutation dans les gènes *rpoB, fabG1, gyrA, Rv1042c, Rv1149*, *embB, pncA* et *katG,* ladite étape comprenant la détermination de la présence des séquences SEQ ID NOs 1, 4, 6, 7, 9, 10 et 12, et de préférence les SEQ ID NOs :1 à 12, dans lesdits gènes respectifs, et une étape (b) de détermination du caractère sensible ou résistant en fonction de présence ou non desdites séquences. Le tableau 7 dans les exemples de réalisation reprend les différentes conclusions quant au caractère sensible ou résistant de ladite souche en fonction de la présence ou non desdites séquences.

Selon un mode de réalisation particulier, la méthode peut comprendre une étape d'obtention du génome de ladite souche *Mycobacterium tuberculosis* préalablement à la détermination de la présence ou absence d'au moins un mutation dans le gène *rpoB,* et optionnellement dans les gènes additionnels.

Au sens de la présente description, le terme « sujet » désigne un être humain et de préférence, le sujet est un patient.

Ainsi, la méthode selon l'invention peut être une méthode *in vitro* ou *ex vivo* pour déterminer la susceptibilité au pyrazinamide d'une souche de l'espèce *Mycobacterium tuberculosis* à partir d'un échantillon biologique d'un sujet susceptible de contenir ladite souche, ladite méthode comprenant les étapes suivantes de :
- détermination de la présence dans le génome de ladite souche d'au moins une mutation dans le gène *rpoB* entre les positions 761112 et 761182, et
- détermination du caractère sensible ou résistant de ladite souche en fonction de la présence ou non de ladite mutation.

Les modes de réalisation spécifiques ou préférés décrits précédemment selon l'invention s'appliquent, bien entendu, aux méthodes *in vitro* ou ex *vivo* faisant également l'objet de l'invention. Ainsi, la détermination d'au moins une mutation dans un des gènes additionnels tels que décrits précédemment est possible.

Par « échantillon biologique », on se réfère ici à tout échantillon provenant d'un sujet, et pouvant être de différentes natures, comme le sang ou ses dérivés ou les expectorations, dans lequel il est possible de trouver des traces de la souche.

Selon un mode de réalisation particulier, l'échantillon biologique est un échantillon d'expectoration, un échantillon de sang ou encore un échantillon dérivé du sang, qui peut notamment être choisi parmi le sang total (tel que collecté par voie veineuse, c'est-à-dire contenant les cellules blanches et rouges, les plaquettes et le plasma), le plasma et le sérum.

### Kit pour mettre en oeuvre la méthode selon l'invention

Des kits peuvent être préparés pour mettre en oeuvre la méthode selon l'un quelconque des modes de réalisation précédents. En particulier, un autre objet de l'invention concerne un kit permettant la détection et/ou l'amplification d'au moins une séquence choisie parmi les séquences SEQ ID NO : 1-3. Le kit peut avantageusement comprendre une notice d'utilisation.

Le kit comprend ainsi des moyens permettant de détecter et/ou amplifier et/ou quantifier lesdites séquences, par exemple dans un échantillon biologique suspecté de contenir une souche de l'espèce *Mycobacterium tuberculosis.* Il peut ainsi s'agir d'amorces et/ou de sondes spécifiques desdites séquences.

Selon un mode de réalisation préféré, le kit comprend des moyens de détection des séquences SEQ ID NO : 1, SEQ ID NO : 2 et SEQ ID NO : 3. De manière avantageuse, le kit contient tous les moyens nécessaires à la mise en oeuvre de la détection des séquences SEQ ID NOs : 1 à 3 par amplification, de préférence par PCR, et notamment par qPCR.

Selon un mode de réalisation particulier, le kit comprend également des moyens, de préférence des amorces et/ou des sondes, permettant de détecter également au moins l'une des séquences SEQ ID NOs : 4 à 12 au sein d'une souche de l'espèce *Mycobacterium tuberculosis.* De préférence, le kit contient des amorces et/ou des sondes pour la détection de l'ensemble des séquences SEQ ID NOs 4 à 12.

L'ensemble des modes de réalisation spécifiques ou préférés décrits précédemment en lien avec les méthodes selon l'invention s'appliquent également au kit faisant l'objet de l'invention.

On entend par « amorce » ou « amorce d'amplification », un fragment nucléotidique pouvant être constitué de 5 à 100 nucléotides, de préférence de 10 à 20 nucléotides, et possédant une spécificité d'hybridation avec une séquence nucléotidique cible, à savoir l'une des séquences SEQ ID NOs 1 à 12, dans des conditions déterminées pour l'initiation d'une polymérisation enzymatique, par exemple dans une réaction d'amplification enzymatique de la séquence nucléotidique cible. Généralement, on utilise des « couples d'amorces », constitués de deux amorces. Lorsque l'on souhaite réaliser l'amplification de plusieurs biomarqueurs (ex. des gènes) différents, plusieurs couples d'amorces différents sont de préférence utilisés, ayant préférentiellement chacun une capacité à s'hybrider spécifiquement avec un biomarqueur différent.

La personne du métier est ainsi en mesure à partir des séquences SEQ ID NOs : 1 à 12, de déterminer les amorces et les sondes nécessaires pour l'amplification.

On entend par « sonde » ou « sonde d'hybridation », un fragment nucléotidique constitué typiquement de 5 à 100 nucléotides, de préférence de 10 à 90 nucléotides, de manière encore plus préférée de 15 à 35 nucléotides, possédant une spécificité d'hybridation dans des conditions déterminées pour former un complexe d'hybridation avec une séquence nucléotidique cible. La sonde comporte également un rapporteur (tel qu'un fluorophore, une enzyme ou tout autre système de détection), qui va permettre la détection de la séquence nucléotidique cible. Dans la présente invention, les séquences nucléotidiques cibles sont les séquences SEQ ID NOs 1 à 3, et de préférence les séquences SEQ ID NOs 1 à 12. Ainsi, plusieurs sondes différentes sont de préférence utilisées, chacune ayant préférentiellement une capacité à s'hybrider spécifiquement avec l'une des séquences cibles.

Par « hybridation », on entend le processus au cours duquel, dans des conditions appropriées, deux fragments nucléotidiques, tels que par exemple une sonde d'hybridation et un fragment nucléotidique cible, ayant des séquences suffisamment complémentaires, sont susceptibles de former un double brin avec des liaisons hydrogènes stables et spécifiques. Un fragment nucléotidique "capable de s'hybrider" avec un polynucléotide est un fragment pouvant s'hybrider avec ledit polynucléotide dans des conditions d'hybridation, qui peuvent être déterminées dans chaque cas de façon connue. Les conditions d'hybridation sont déterminées par la stringence, c'est-à-dire la rigueur des conditions opératoires. L'hybridation est d'autant plus spécifique qu'elle est effectuée à plus forte stringence. La stringence est définie notamment en fonction de la composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier.

En général, selon la longueur des sondes d'hybridation utilisées, la température pour la réaction d'hybridation est comprise entre environ 20 et 70°C, en particulier entre 35 et 65°C dans une solution saline à une concentration d'environ 0,5 à 1 M. On réalise ensuite une étape de détection de la réaction d'hybridation.

Les sondes ou amorces qui peuvent être utilisées dans les méthodes de l'invention peuvent être typiquement des molécules d'acide nucléique courtes, par exemple des oligonucléotides d'ADN de 10 nucléotides ou plus de longueur, qui peuvent être liées à la molécule d'acide nucléique cible complémentaire par hybridation d'acide nucléique pour former un hybride entre l'amorce ou la sonde et le brin d'acide nucléique cible.

La sonde ou les amorces peuvent être non marquées ou marquées de façon à ce que leur liaison à une séquence cible puisse être détectée (par exemple, avec un marqueur donneur ou accepteur de type FRET).

Une amorce peut être étendue le long de la molécule d'acide nucléique cible par une enzyme polymérase.

Par conséquent, des amorces peuvent être utilisées pour amplifier la molécule d'acide nucléique cible, telle que l'une des séquences SEQ ID NOs :1 à 12, et/ou leurs séquences variantes.

La spécificité d'une sonde ou d'une amorce augmente avec sa longueur. Ainsi, par exemple, une sonde ou une amorce qui comprend 30 nucléotides consécutifs se fixera à une séquence cible avec une plus grande spécificité qu'une amorce correspondante de seulement 15 nucléotides. Ainsi, pour obtenir une plus grande spécificité, on peut choisir des sondes et des amorces qui comprennent au moins 15, 20, 25, 30, 35, 40, 45, 50, 55, 60, 65, 70 ou plus de nucléotides consécutifs.

Dans des exemples particuliers, une amorce peut avoir une longueur d'au moins 15 nucléotides, comme par exemple au moins 15 nucléotides contigus complémentaires à une molécule d'acide nucléique cible. Les longueurs particulières d'amorces qui peuvent être utilisées pour pratiquer les méthodes de la présente divulgation comprennent des amorces ayant au moins 15, au moins 16, au moins 17, au moins 18, au moins 19, au moins 20, au moins 21, au moins 22, au moins 23, au moins 24, au moins 25, au moins 26, au moins 27, au moins 28, au moins 29, au moins 30, au moins 31, au moins 32, au moins 33, au moins 34, au moins 35, au moins 36, au moins 37, au moins 38, au moins 39, au moins 40, au moins 45, au moins 50, au moins 55, au moins 60, au moins 65, au moins 70, ou plus de nucléotides contigus complémentaires à la molécule d'acide nucléique cible à amplifier, telle qu'une amorce de 15-70 nucléotides, 15-60 nucléotides, 15-50 nucléotides, ou 15-30 nucléotides.

Une amorce "sens" (dite également forward) est une amorce située en 5' d'un point de référence sur une séquence d'acide nucléique. Une amorce "anti-sens" (dite également reverse) est une amorce située en 3' d'un point de référence sur une séquence d'acide nucléique. En général, au moins une amorce sens et une amorce anti-sens (la "paire d'amorces") sont incluses dans une réaction d'amplification.

Les sondes et les amorces d'acide nucléique, ou les paires d'amorces, peuvent être facilement préparées sur la base de la séquence d'acide nucléique de n'importe laquelle des séquences SEQ ID NOs 1-12. Les paires d'amorces PCR peuvent être dérivées desdites séquences en utilisant des programmes informatiques prévus à cet effet tels que Primer 3 (v. 0.4.0 Whitehead Institute for Biomedical Research, Steve Rozen, and Helen Skaletsky).

Selon un mode de réalisation particulier, les séquences SEQ ID NOs 1-12, ou leurs produits amplifiés ou transformés (ADNc par exemple) sont détectés par hybridation spécifique de sondes d'acide nucléique.

Ces sondes peuvent également être immobilisées sur une surface solide (telle que la nitrocellulose, le verre, le quartz, une lame de silice fondue) comme dans un réseau, un microréseau ou une puce à ADN. La personne du métier est en mesure de reconnaitre que la séquence précise de sondes et d'amorces particulières peut être modifiée à partir de la séquence cible jusqu'à un certain degré pour produire des sondes qui sont "substantiellement identiques" ou "substantiellement complémentaires" à une séquence cible, tout en conservant la capacité de se lier spécifiquement (c'est-à-dire de s'hybrider spécifiquement) aux mêmes cibles dont elles sont dérivées.

Dans le contexte de la présente description, les termes "capable de s'hybrider à" et "se lie spécifiquement à", qui sont utilisés de manière interchangeable, font référence à une séquence polynucléotidique qui forme des liaisons Watson-Crick avec une séquence complémentaire. En fonction de la longueur des polynucléotides, de la longueur de la région complémentaire et de la rigueur des conditions, la personne du métier comprend que le pourcentage de complémentarité ne doit pas nécessairement être de 100 % pour que l'hybridation ou la liaison spécifique se produise. Par exemple, une amorce ou une sonde est au moins 60 %, 70 %, 80 %, 90 %, 95 %, 99 % ou 100 % complémentaire sur la longueur de la région complémentaire.

Un autre objet de l'invention concerne l'utilisation du kit tel que défini précédemment pour la détermination de la susceptibilité d'une souche *Mycobacterium tuberculosis* au pyrazinamide.

Un autre objet de l'invention concerne l'utilisation des séquences SEQ ID NOs 1 à 3, et de préférence également les séquences SEQ ID NOs 4 à 12, pour déterminer la susceptibilité d'une souche *Mycobacterium tuberculosis* au pyrazinamide.

Les modes de réalisation spécifiques ou préférés décrits en lien avec les méthodes selon l'invention s'appliquent, bien entendu, aux utilisations faisant l'objet de l'invention.

### Mise en oeuvre par ordinateur

La méthode telle que décrite selon n'importe quel mode de réalisation détaillé précédemment peut tout à fait être mise en oeuvre par ordinateur. Dans ce cas, elle peut être exécutée, partiellement ou totalement, par des moyens informatiques.

A cette fin, l'invention prévoir également un appareil de traitement de donnée comprenant :
(a) des moyens pour mettre en application une méthode mis en oeuvre par ordinateur selon l'invention, notamment des moyens pour déterminer la présence de mutations dans le gène *rpoB* de la souche *Mycobacterium tuberculosis,* et/ou des moyens permettant de comparer les séquences desdits gènes avec le génome de référence *Mycobacterium tuberculosis* H37Rv (référence NC_000962.3), et/ou des moyens pour identifier la présence des séquences SEQ ID NOs 1 à 3 dans le génome de la souche *Mycobacterium tuberculosis,* et/ou des moyens pour fournir une donnée de sortie de la présence ou de l'absence desdites séquences, lorsque lesdits moyens sont mis en oeuvre ou pilotés par ordinateur
(b) un processeur adapté à ou configuré pour exécuter une méthode mis en oeuvre par ordinateur selon l'invention, en particulier un processeur adapté à ou configuré pour exécuter les étapes d'une méthode mise en oeuvre par ordinateur selon l'invention.

De manière avantageuse, l'appareil de traitement peut également comprendre des moyens pour déterminer la présence de mutations dans les gènes *fabG1, gyrA, Rv1042c, Rv1149, embB, pncA* et *katG* aux positions telles que définies précédemment, et/ou des moyens pour identifier la présence ou l'absence des séquences SEQ ID NOs :4 à 12 dans lesdits gènes auxquels elles se rapportent.

Selon un mode de réalisation particulier, un tel appareil de traitement des données comprend :
(a) une interface d'entrée pour recevoir le génome d'une souche *Mycobacterium tuberculosis* dont on cherche à déterminer la susceptibilité au pyrazinamide,
(b) une mémoire pour stocker au moins des instructions d'un programme informatique qui, lorsque le programme est exécuté par un ordinateur ou un processeur, amène à l'exécution de la détermination de la présence de mutations dans le gène *rpoB* aux positions décrites précédemment, et éventuellement dans un ou plusieurs gènes additionnels tels que décrits précédemment,
c) un processeur accédant à la mémoire pour lire les instructions susmentionnées et exécuter une méthode mis en oeuvre par ordinateur selon l'invention
(d) une interface de sortie pour fournir les valeurs de sortie, en particulier les valeurs de sortie correspondant à la conclusion du caractère sensible ou résistant au pyrazinamide de la souche.

### Autres objets de la description

Un autre objet de la présente description concerne une méthode de traitement d'un sujet infecté par une souche de l'espèce *Mycobacterium tuberculosis* comprenant une étape de détermination de la susceptibilité de ladite souche au pyrazinamide selon l'une quelconque des méthodes telles que décrites précédemment, et une étape de traitement au pyrazinamide dudit sujet lorsqu'il est conclu au caractère sensible de ladite souche.

En particulier, le traitement peut être initié aussitôt qu'il est conclu au caractère sensible.

Un autre objet de la présente description concerne un méthode comprenant les étapes suivantes :
- obtenir un échantillon biologique d'un sujet infecté par une souche de l'espèce *Mycobacterium tuberculosis,*
- mettre en contact ledit échantillon biologique avec des moyens de détection ou des réactifs spécifiques permettant de détecter la présence de mutations dans le gène *rpoB* de ladite souche entre les positions 761112 et 761182, et
- déterminer la présence des séquences SEQ ID NOs :1 à 3.

Les réactifs spécifiques des produits d'expression sont sélectionnés parmi des amorces d'amplification, des sondes d'hybridation et sont tels que définis précédemment, et permettent notamment de déterminer la présence des séquences SEQ ID NOs : 1 à 3.

De préférence, l'échantillon biologique est également mis en contact avec des moyens pour déterminer la présence de mutations dans les gènes *fabG1, gyrA, Rv1042c, Rv1149, embB, pncA* et *katG* aux positions telles que définies précédemment et permet notamment d'identifier la présence ou l'absence des séquences SEQ ID NOs :4 à 12 dans lesdits gènes auxquels elles se rapportent.

La présente invention est illustrée de manière non limitative à partir des exemples ci-dessous.

### EXEMPLES

### Exemple 1 : détermination de la susceptibilité d'une souche de Mycobacterium tuberculosis au pyrazinamide.

### 1. Obtention des modèles

Un ensemble de 3606 génomes de *Mycobacterium tuberculosis* possédant des phénotypes résistant (R) ou sensible (S) au pyrazinamide a été utilisé. Parmi ces génomes, 3038 ont été utilisés pour entrainer les modèles d'apprentissages automatiques supervisés (set d'apprentissage) et 568 pour évaluer les performances de ces modèles (set de validation).

Au sein des 568 génomes du set de validation, 133 sont résistants au pyrazinamide et 435 y sont sensibles. Les tableaux ci-après présentent pour chaque modèle la présence de chaque séquence « Wild type » du modèle dans ces génomes de validation. Les séquences sont nommées par le nom du gène auxquelles elles appartiennent et sont ordonnées par valeur décroissante du coefficient dans le modèle (donné en valeur absolue, entre parenthèses).

Les tableaux ci-après mettent en évidence différents haplotypes définis comme des combinaisons de séquences mutées ou non mutées observées. Les haplotypes sont ordonnés par prévalence décroissante (pourcentage d'observations parmi les 568 génomes).

Pour chaque haplotype, il est reporté la prédiction faite par le modèle, ainsi que le nombre de génomes résistants et sensibles dans lesquels cet haplotype a été observé. Ainsi, il est possible d'identifier le nombre de bonnes prédictions et le nombre d'erreurs faites par le modèle dans le set de validation.

Ces erreurs sont décrites en termes de « Very Major Error» (VME) correspondant à un génome résistant prédit sensible par le modèle (autrement dit, un faux négatif) et de « Major Error » (ME) correspondant à un génome sensible prédit résistant par le modèle (autrement dit, un faux positif).

Légendes des tableaux 1 à 6: NbSR = nombre de souches résistantes ; NbSS = nombre de souches sensible ; Prev = prévalence ; Pred = prédiction ; 1 : présence de la séquence ; 0 : absence de la séquence.

### 2. Application des modèles pour la détermination de la susceptibilité au pyrazinamide

### 2.1 Modèle rpoB

Le tableau 1 ci-dessous présente les résultats de la susceptibilité par détection de la présence de mutations dans le gène *rpoB* de la souche par la mise en évidence de la présence ou de l'absence des séquences SEQ ID NOs : 1 à 3.

**Tableau 1**

| | | | | | | **SEQ ID NO** : | | |
|---|---|---|---|---|---|---|---|---|
| | | | | | | **2** | **1** | **3** |
| **NbSR** | **NbSS** | **Prev** | **Pred** | **ME** | **VME** | **rpoB (5.3)** | **rpoB (4.33)** | **rpoB (0.94)** |
| 7 | 391 | 70,07 | S | 0 | 7 | 1 | 1 | 1 |
| 98 | 23 | 21,3 | R | 23 | 0 | 0 | 1 | 0 |
| 19 | 4 | 4,05 | R | 4 | 0 | 1 | 0 | 1 |
| 3 | 13 | 2,82 | S | 0 | 3 | 1 | 0 | 0 |
| 5 | 3 | 1,41 | S | 0 | 5 | 1 | 1 | 0 |
| 1 | 0 | 0,18 | R | 0 | 0 | 0 | 0 | 0 |

### Interprétation des résultats

Dans ce modèle, l'haplotype majoritaire est celui dans lequel on observe la présence des séquences SEQ ID NOs : 1 à 3. Cet haplotype est observé dans 398 génomes (391+7), soit 70,07% des 568 génomes de validation. Par conséquent, la détermination de la présence des séquences SEQ ID NOs : 1 à 3 conduit à la bonne détermination (ou prédiction) du caractère « sensible » pour 391 génomes, mais avec 7 VME.

A l'inverse, l'absence desdites séquences SEQ ID NOs : 1 à 3 représente l'haplotype minoritaire et il est observé dans un seul génome, soit 0,18% des génomes de validation. Par conséquent, la détermination de l'absence des séquences SEQ ID NOs : 1 à 3 conduit à la bonne détermination (ou prédiction) du caractère « résistant » sans ME ou VME.

### Performances du modèle

Au global, ce modèle permet de prédire sur la base de la présence ou de l'absence des séquences SEQ ID NOs : 1 à 3, le caractère sensible ou résistant d'une souche *Mycobacterium tuberculosis* avec une spécificité de 88,72%, une sensibilité de 93,56% et un taux d'erreur à 7,57% (ME et VME).

### 2.2 Modèle rpoB-fabG1

Le tableau 2 ci-dessous présente les résultats de la susceptibilité par détection de la présence de mutations dans les gènes *rpoB* et *fabG1* de la souche par la mise en évidence de la présence ou absence des séquences SEQ ID NOs : 1 à 5.

**Tableau 2**

| | | | | | | **SEQ ID NO** : | | | | |
|---|---|---|---|---|---|---|---|---|---|---|
| | | | | | | **2** | **1** | **4** | **5** | **3** |
| **NbSR** | **NbSS** | **Prev** | **Pred** | **ME** | **VME** | **rpoB (4.99)** | **rpoB (4.1)** | **fabG1 (1.02)** | **fabG1 (1.02)** | **rpoB (0.79)** |
| 6 | 359 | 64,26 | S | 0 | 6 | 1 | 1 | 1 | 1 | 1 |
| 44 | 19 | 11,09 | R | 19 | 0 | 0 | 1 | 1 | 1 | 0 |
| 54 | 4 | 10,21 | R | 4 | 0 | 0 | 1 | 0 | 0 | 0 |
| 1 | 32 | 5,81 | S | 0 | 1 | 1 | 1 | 0 | 0 | 1 |
| 1 | 13 | 2,46 | S | 0 | 1 | 1 | 0 | 1 | 1 | 0 |
| 8 | 4 | 2,11 | R | 4 | 0 | 1 | 0 | 1 | 1 | 1 |
| 11 | 0 | 1,94 | R | 0 | 0 | 1 | 0 | 0 | 0 | 1 |
| 2 | 3 | 0,88 | S | 0 | 2 | 1 | 1 | 1 | 1 | 0 |
| 3 | 0 | 0,53 | S | 0 | 3 | 1 | 1 | 0 | 0 | 0 |
| 2 | 0 | 0,35 | R | 0 | 0 | 1 | 0 | 0 | 0 | 0 |
| 1 | 0 | 0,18 | R | 0 | 0 | 0 | 0 | 1 | 1 | 0 |
| 0 | 1 | 0,18 | R | 1 | 0 | 0 | 1 | 1 | 1 | 1 |

### Interprétation des résultats

Dans ce modèle, l'haplotype majoritaire est celui dans lequel on observe la présence des séquences SEQ ID NOs : 1 à 5. Cet haplotype est observé dans 365 génomes (359+6), soit 64,26% des 568 génomes de validation. Par conséquent, la détermination de la présence des séquences SEQ ID NOs : 1 à 5 conduit à la bonne détermination (ou prédiction) du caractère « sensible » pour 359 génomes, mais avec 6 VME.

### Performances du modèle

Au global, ce modèle permet de prédire sur la base de la présence ou de l'absence des séquences SEQ ID NOs : 1 à 5, le caractère sensible ou résistant d'une souche *Mycobacterium tuberculosis* avec une spécificité de 90,23 %, une sensibilité de 93,56% et un taux d'erreur à 7,21% (ME et VME).

### 2.3 Modèle rpoB-fabG1-gyrA

Le tableau 3 ci-dessous présente les résultats de la susceptibilité par détection de la présence de mutations dans les gènes *rpoB, fabG1* et *gyrA* de la souche par la mise en évidence de la présence ou absence des séquences SEQ ID NOs : 1 à 6.

**Tableau 3**

| | | | | | | **SEQ ID NO** : | | | | | |
|---|---|---|---|---|---|---|---|---|---|---|---|
| | | | | | | **2** | **1** | **6** | **4** | **5** | **3** |
| **NbSR** | **NbSS** | **Prev** | **Pred** | **ME** | **VME** | **rpoB (4.47)** | **rpoB (3.62)** | **gyrA (1.85)** | **fabG1 (0.56)** | **fabG1 (0.56)** | **rpoB (0.52)** |
| 5 | 357 | 63,73 | S | 0 | 5 | 1 | 1 | 1 | 1 | 1 | 1 |
| 42 | 1 | 7,57 | R | 1 | 0 | 0 | 1 | 0 | 0 | 0 | 0 |
| 22 | 19 | 7,22 | R | 19 | 0 | 0 | 1 | 1 | 1 | 1 | 0 |
| 0 | 32 | 5,63 | S | 0 | 0 | 1 | 1 | 1 | 0 | 0 | 1 |
| 22 | 0 | 3,87 | R | 0 | 0 | 0 | 1 | 0 | 1 | 1 | 0 |
| 12 | 3 | 2,64 | R | 3 | 0 | 0 | 1 | 1 | 0 | 0 | 0 |
| 1 | 13 | 2,46 | S | 0 | 1 | 1 | 0 | 1 | 1 | 1 | 0 |
| 9 | 0 | 1,58 | R | 0 | 0 | 1 | 0 | 0 | 0 | 0 | 1 |
| 7 | 1 | 1,41 | R | 1 | 0 | 1 | 0 | 0 | 1 | 1 | 1 |
| 1 | 3 | 0,7 | S | 0 | 1 | 1 | 0 | 1 | 1 | 1 | 1 |
| 1 | 3 | 0,7 | S | 0 | 1 | 1 | 1 | 1 | 1 | 1 | 0 |
| 1 | 2 | 0,53 | S | 0 | 1 | 1 | 1 | 0 | 1 | 1 | 1 |
| 2 | 0 | 0,35 | R | 0 | 0 | 1 | 0 | 1 | 0 | 0 | 1 |
| 2 | 0 | 0,35 | S | 0 | 2 | 1 | 1 | 1 | 0 | 0 | 0 |
| 1 | 0 | 0,18 | R | 0 | 0 | 0 | 0 | 1 | 1 | 1 | 0 |
| 0 | 1 | 0,18 | R | 1 | 0 | 0 | 1 | 1 | 1 | 1 | 1 |
| 1 | 0 | 0,18 | R | 0 | 0 | 1 | 0 | 0 | 0 | 0 | 0 |
| 1 | 0 | 0,18 | S | 0 | 1 | 1 | 0 | 1 | 0 | 0 | 0 |
| 1 | 0 | 0,18 | S | 0 | 1 | 1 | 1 | 0 | 0 | 0 | 0 |
| 1 | 0 | 0,18 | S | 0 | 1 | 1 | 1 | 0 | 0 | 0 | 1 |
| 1 | 0 | 0,18 | S | 0 | 1 | 1 | 1 | 0 | 1 | 1 | 0 |

### Interprétation des résultats

Dans ce modèle, l'haplotype majoritaire est celui dans lequel on observe la présence des séquences SEQ ID NOs : 1 à 6. Cet haplotype est observé dans 362 génomes (357+5), soit 63,73% des 568 génomes de validation. Par conséquent, la détermination de la présence des séquences SEQ ID NOs : 1 à 6 conduit à la bonne détermination (ou prédiction) du caractère « sensible » pour 357 génomes, mais avec 5 VME.

### Performances du modèle

Au global, ce modèle permet de prédire sur la base de la présence ou de l'absence des séquences SEQ ID NOs : 1 à 6, le caractère sensible ou résistant d'une souche *Mycobacterium tuberculosis* avec une spécificité de 94,25%, une sensibilité de 88,72% et un taux d'erreur à 7,04% (ME et VME).

### 2.4 Modèle rpoB-fabG1-gyrA-Rv1042c/Rv1149

Le tableau 4 ci-dessous présente les résultats de la susceptibilité par détection de la présence de mutations dans les gènes rpoB, fabG1, gyrA, *Rv1042c* et *Rv1149* de la souche par la mise en évidence de la présence ou absence des séquences SEQ ID NOs : 1 à 8.

**Tableau 4**

| | | | | | | **SEQ ID NO** : | | | | | | | |
|---|---|---|---|---|---|---|---|---|---|---|---|---|---|
| | | | | | | **2** | **1** | **6** | **7** | **4** | **5** | **8** | **3** |
| **NbSR** | **NbSS** | **Prev** | **Pred** | **ME** | **VME** | **rpoB (3.99)** | **rpoB (3.07)** | **gyrA (1.8)** | **Rv1042c /Rv1149 (1.24)** | **fabG1 (0.4)** | **fabG1 (0.4)** | **Rv1042c /Rv1149 (0.61)** | **rpoB (0.48)** |
| 1 | 186 | 32,92 | S | 0 | 1 | 1 | 1 | 1 | 1 | 1 | 1 | 0 | 1 |
| 1 | 85 | 15,14 | S | 0 | 1 | 1 | 1 | 1 | 0 | 1 | 1 | 0 | 1 |
| 3 | 82 | 14,96 | S | 0 | 3 | 1 | 1 | 1 | 1 | 1 | 1 | 1 | 1 |
| 37 | 1 | 6,69 | R | 1 | 0 | 0 | 1 | 0 | 1 | 0 | 0 | 1 | 0 |
| 15 | 7 | 3,87 | R | 7 | 0 | 0 | 1 | 1 | 1 | 1 | 1 | 1 | 0 |
| 0 | 19 | 3,35 | S | 0 | 0 | 1 | 1 | 1 | 1 | 0 | 0 | 0 | 1 |
| 18 | 0 | 3,17 | R | 0 | 0 | 0 | 1 | 0 | 1 | 1 | 1 | 1 | 0 |
| 6 | 9 | 2,64 | R | 9 | 0 | 0 | 1 | 1 | 1 | 1 | 1 | 0 | 0 |
| 11 | 0 | 1,94 | R | 0 | 0 | 0 | 1 | 1 | 1 | 0 | 0 | 1 | 0 |
| 9 | 0 | 1,58 | R | 0 | 0 | 1 | 0 | 0 | 1 | 0 | 0 | 1 | 1 |
| 0 | 7 | 1,23 | S | 0 | 0 | 1 | 0 | 1 | 1 | 1 | 1 | 0 | 0 |
| 0 | 7 | 1,23 | S | 0 | 0 | 1 | 1 | 1 | 1 | 0 | 0 | 1 | 1 |
| 6 | 0 | 1,06 | R | 0 | 0 | 1 | 0 | 0 | 1 | 1 | 1 | 1 | 1 |
| 1 | 5 | 1,06 | S | 0 | 1 | 1 | 0 | 1 | 1 | 1 | 1 | 1 | 0 |
| 0 | 6 | 1,06 | S | 0 | 0 | 1 | 1 | 1 | 0 | 0 | 0 | 0 | 1 |
| 5 | 0 | 0,88 | R | 0 | 0 | 0 | 1 | 0 | 1 | 0 | 0 | 0 | 0 |
| 1 | 3 | 0,7 | S | 0 | 1 | 0 | 1 | 1 | 0 | 1 | 1 | 0 | 0 |
| 1 | 3 | 0,7 | R | 3 | 0 | 0 | 1 | 1 | 1 | 0 | 0 | 0 | 0 |
| 0 | 4 | 0,7 | S | 0 | 0 | 1 | 1 | 1 | 0 | 1 | 1 | 1 | 1 |
| 3 | 0 | 0,53 | R | 0 | 0 | 0 | 1 | 0 | 1 | 1 | 1 | 0 | 0 |
| 1 | 2 | 0,53 | R | 2 | 0 | 1 | 0 | 1 | 1 | 1 | 1 | 1 | 1 |
| 1 | 2 | 0,53 | S | 0 | 1 | 1 | 1 | 1 | 1 | 1 | 1 | 0 | 0 |
| 1 | 1 | 0,35 | R | 1 | 0 | 1 | 0 | 0 | 1 | 1 | 1 | 0 | 1 |
| 2 | 0 | 0,35 | R | 0 | 0 | 1 | 0 | 1 | 1 | 0 | 0 | 1 | 1 |
| 1 | 1 | 0,35 | S | 0 | 1 | 1 | 1 | 0 | 1 | 1 | 1 | 0 | 1 |
| 1 | 0 | 0,18 | R | 0 | 0 | 0 | 0 | 1 | 1 | 1 | 1 | 1 | 0 |
| 1 | 0 | 0,18 | R | 0 | 0 | 0 | 1 | 0 | 0 | 1 | 1 | 0 | 0 |
| 0 | 1 | 0,18 | S | 0 | 0 | 0 | 1 | 1 | 0 | 1 | 1 | 0 | 1 |
| 1 | 0 | 0,18 | R | 0 | 0 | 1 | 0 | 0 | 1 | 0 | 0 | 0 | 0 |
| 0 | 1 | 0,18 | S | 0 | 0 | 1 | 0 | 1 | 0 | 1 | 1 | 0 | 0 |
| 1 | 0 | 0,18 | S | 0 | 1 | 1 | 0 | 1 | 1 | 0 | 0 | 0 | 0 |
| 0 | 1 | 0,18 | S | 0 | 0 | 1 | 0 | 1 | 1 | 1 | 1 | 0 | 1 |
| 1 | 0 | 0,18 | S | 0 | 1 | 1 | 1 | 0 | 1 | 0 | 0 | 0 | 0 |
| 1 | 0 | 0,18 | S | 0 | 1 | 1 | 1 | 0 | 1 | 0 | 0 | 1 | 1 |
| 1 | 0 | 0,18 | S | 0 | 1 | 1 | 1 | 0 | 1 | 1 | 1 | 1 | 0 |
| 0 | 1 | 0,18 | S | 0 | 0 | 1 | 1 | 0 | 1 | 1 | 1 | 1 | 1 |
| 1 | 0 | 0,18 | S | 0 | 1 | 1 | 1 | 1 | 1 | 0 | 0 | 0 | 0 |
| 1 | 0 | 0,18 | S | 0 | 1 | 1 | 1 | 1 | 1 | 0 | 0 | 1 | 0 |
| 0 | 1 | 0,18 | S | 0 | 0 | 1 | 1 | 1 | 1 | 1 | 1 | 1 | 0 |

### Interprétation des résultats

Dans ce modèle, l'haplotype majoritaire est celui dans lequel on détecte la présence des séquences SEQ ID NOs : 1 à 6 et SEQ ID NO : 7 et l'absence de la séquence SEQ ID NO : 8. Cet haplotype est observé dans 187 génomes (186+1), soit 32,92% des 568 génomes de validation.

Par conséquent, la détermination de la présence des séquences SEQ ID NOs : 1 à 6 et SEQ ID NO : 7 et de l'absence de la séquence SEQ ID NO :8, conduit à la bonne détermination (ou prédiction) du caractère « sensible » pour 186 génomes et avec 1 seule VME.

De la même manière, l'haplotype dans lequel on détecte la présence des séquences SEQ ID NOs : 1 à 6 et l'absence des séquences SEQ ID NO : 7 et 8 est observé dans 86 génomes, soit 14,14% des génomes de validation. La détermination de la présence et de l'absence desdites séquences permet ainsi de prédire le caractère « sensible » pour 85 génomes et avec également 1 seule VME.

### Performances du modèle

Au global, ce modèle permet de prédire sur la base de la présence ou de l'absence des séquences SEQ ID NOs : 1 à 8, le caractère sensible ou résistant d'une souche *Mycobacterium tuberculosis* avec une spécificité de 88,72%, une sensibilité de 94,71% et un taux d'erreur à 6,69% (ME et VME).

### 2.5 Modèle rpoB-fabG1-gyrA-Rv1042c/Rv1149-embB

Le tableau 5 ci-dessous présente les résultats de la susceptibilité par détection de la présence de mutations dans les gènes *rpoB, fabG1, gyrA, Rv1042c, Rv1149* et *embB* de la souche par la mise en évidence de la présence ou absence des séquences SEQ ID NOs : 1 à 9.

**Tableau 5**

| | | | | | | **SEQ ID NO** : | | | | | | | | |
|---|---|---|---|---|---|---|---|---|---|---|---|---|---|---|
| | | | | | | **2** | **1** | **6** | **7** | **4** | **5** | **8** | **9** | **3** |
| **NbSR** | **NbSS** | **Prev** | **Pred** | **ME** | **VME** | **rpoB (3.2)** | **rpoB (2.57)** | **gyrA (1.63)** | **Rv1042c/ Rv1149 (1.19)** | **fabG1 (0.44)** | **fabG1 (0.44)** | **Rv1042c/ Rv1149 (0.74)** | **embB (1.27)** | **rpoB (0.35)** |
| 1 | 185 | 32,75 | S | 0 | 1 | 1 | 1 | 1 | 1 | 1 | 1 | 0 | 1 | 1 |
| 1 | 84 | 14,96 | S | 0 | 1 | 1 | 1 | 1 | 0 | 1 | 1 | 0 | 1 | 1 |
| 3 | 82 | 14,96 | S | 0 | 3 | 1 | 1 | 1 | 1 | 1 | 1 | 1 | 1 | 1 |
| 31 | 0 | 5,46 | R | 0 | 0 | 0 | 1 | 0 | 1 | 0 | 0 | 1 | 0 | 0 |
| 0 | 19 | 3,35 | S | 0 | 0 | 1 | 1 | 1 | 1 | 0 | 0 | 0 | 1 | 1 |
| 10 | 3 | 2,29 | R | 3 | 0 | 0 | 1 | 1 | 1 | 1 | 1 | 1 | 1 | 0 |
| 4 | 7 | 1,94 | S | 0 | 4 | 0 | 1 | 1 | 1 | 1 | 1 | 0 | 1 | 0 |
| 10 | 0 | 1,76 | R | 0 | 0 | 0 | 1 | 0 | 1 | 1 | 1 | 1 | 0 | 0 |
| 10 | 0 | 1,76 | R | 0 | 0 | 0 | 1 | 1 | 1 | 0 | 0 | 1 | 0 | 0 |
| 5 | 4 | 1,58 | R | 4 | 0 | 0 | 1 | 1 | 1 | 1 | 1 | 1 | 0 | 0 |
| 9 | 0 | 1,58 | R | 0 | 0 | 1 | 0 | 0 | 1 | 0 | 0 | 1 | 0 | 1 |
| 8 | 0 | 1,41 | R | 0 | 0 | 0 | 1 | 0 | 1 | 1 | 1 | 1 | 1 | 0 |
| 6 | 1 | 1,23 | R | 1 | 0 | 0 | 1 | 0 | 1 | 0 | 0 | 1 | 1 | 0 |
| 0 | 7 | 1,23 | S | 0 | 0 | 1 | 0 | 1 | 1 | 1 | 1 | 0 | 1 | 0 |
| 0 | 6 | 1,06 | S | 0 | 0 | 1 | 1 | 1 | 0 | 0 | 0 | 0 | 1 | 1 |
| 0 | 6 | 1,06 | S | 0 | 0 | 1 | 1 | 1 | 1 | 0 | 0 | 1 | 1 | 1 |
| 5 | 0 | 0,88 | R | 0 | 0 | 1 | 0 | 0 | 1 | 1 | 1 | 1 | 1 | 1 |
| 4 | 0 | 0,7 | R | 0 | 0 | 0 | 1 | 0 | 1 | 0 | 0 | 0 | 1 | 0 |
| 2 | 2 | 0,7 | R | 2 | 0 | 0 | 1 | 1 | 1 | 1 | 1 | 0 | 0 | 0 |
| 0 | 4 | 0,7 | S | 0 | 0 | 1 | 1 | 1 | 0 | 1 | 1 | 1 | 1 | 1 |
| 3 | 0 | 0,53 | R | 0 | 0 | 0 | 1 | 0 | 1 | 1 | 1 | 0 | 0 | 0 |
| 0 | 3 | 0,53 | S | 0 | 0 | 0 | 1 | 1 | 0 | 1 | 1 | 0 | 1 | 0 |
| 0 | 3 | 0,53 | S | 0 | 0 | 0 | 1 | 1 | 1 | 0 | 0 | 0 | 1 | 0 |
| 0 | 3 | 0,53 | R | 3 | 0 | 1 | 0 | 1 | 1 | 1 | 1 | 1 | 0 | 0 |
| 1 | 2 | 0,53 | S | 0 | 1 | 1 | 0 | 1 | 1 | 1 | 1 | 1 | 1 | 0 |
| 1 | 1 | 0,35 | R | 1 | 0 | 1 | 0 | 0 | 1 | 1 | 1 | 0 | 0 | 1 |
| 1 | 1 | 0,35 | R | 1 | 0 | 1 | 0 | 1 | 1 | 1 | 1 | 1 | 0 | 1 |
| 1 | 1 | 0,35 | S | 0 | 1 | 1 | 1 | 1 | 1 | 1 | 1 | 0 | 0 | 0 |
| 1 | 0 | 0,18 | R | 0 | 0 | 0 | 0 | 1 | 1 | 1 | 1 | 1 | 1 | 0 |
| 1 | 0 | 0,18 | R | 0 | 0 | 0 | 1 | 0 | 0 | 1 | 1 | 0 | 0 | 0 |
| 1 | 0 | 0,18 | R | 0 | 0 | 0 | 1 | 0 | 1 | 0 | 0 | 0 | 0 | 0 |
| 1 | 0 | 0,18 | S | 0 | 1 | 0 | 1 | 1 | 0 | 1 | 1 | 0 | 0 | 0 |
| 0 | 1 | 0,18 | S | 0 | 0 | 0 | 1 | 1 | 0 | 1 | 1 | 0 | 1 | 1 |
| 1 | 0 | 0,18 | R | 0 | 0 | 0 | 1 | 1 | 1 | 0 | 0 | 0 | 0 | 0 |
| 1 | 0 | 0,18 | R | 0 | 0 | 0 | 1 | 1 | 1 | 0 | 0 | 1 | 1 | 0 |
| 1 | 0 | 0,18 | R | 0 | 0 | 1 | 0 | 0 | 1 | 0 | 0 | 0 | 1 | 0 |
| 1 | 0 | 0,18 | R | 0 | 0 | 1 | 0 | 0 | 1 | 1 | 1 | 1 | 0 | 1 |
| 0 | 1 | 0,18 | S | 0 | 0 | 1 | 0 | 1 | 0 | 1 | 1 | 0 | 1 | 0 |
| 1 | 0 | 0,18 | R | 0 | 0 | 1 | 0 | 1 | 1 | 0 | 0 | 0 | 0 | 0 |
| 1 | 0 | 0,18 | R | 0 | 0 | 1 | 0 | 1 | 1 | 0 | 0 | 1 | 0 | 1 |
| 1 | 0 | 0,18 | R | 0 | 0 | 1 | 0 | 1 | 1 | 0 | 0 | 1 | 1 | 1 |
| 0 | 1 | 0,18 | S | 0 | 0 | 1 | 0 | 1 | 1 | 1 | 1 | 0 | 1 | 1 |
| 0 | 1 | 0,18 | S | 0 | 0 | 1 | 0 | 1 | 1 | 1 | 1 | 1 | 1 | 1 |
| 1 | 0 | 0,18 | S | 0 | 1 | 1 | 1 | 0 | 1 | 0 | 0 | 0 | 1 | 0 |
| 1 | 0 | 0,18 | R | 0 | 0 | 1 | 1 | 0 | 1 | 0 | 0 | 1 | 0 | 1 |
| 1 | 0 | 0,18 | S | 0 | 1 | 1 | 1 | 0 | 1 | 1 | 1 | 0 | 0 | 1 |
| 0 | 1 | 0,18 | S | 0 | 0 | 1 | 1 | 0 | 1 | 1 | 1 | 0 | 1 | 1 |
| 1 | 0 | 0,18 | S | 0 | 1 | 1 | 1 | 0 | 1 | 1 | 1 | 1 | 0 | 0 |
| 0 | 1 | 0,18 | S | 0 | 0 | 1 | 1 | 0 | 1 | 1 | 1 | 1 | 1 | 1 |
| 0 | 1 | 0,18 | S | 0 | 0 | 1 | 1 | 1 | 0 | 1 | 1 | 0 | 0 | 1 |
| 1 | 0 | 0,18 | S | 0 | 1 | 1 | 1 | 1 | 1 | 0 | 0 | 0 | 0 | 0 |
| 1 | 0 | 0,18 | S | 0 | 1 | 1 | 1 | 1 | 1 | 0 | 0 | 1 | 0 | 0 |
| 0 | 1 | 0,18 | S | 0 | 0 | 1 | 1 | 1 | 1 | 0 | 0 | 1 | 0 | 1 |
| 0 | 1 | 0,18 | S | 0 | 0 | 1 | 1 | 1 | 1 | 1 | 1 | 0 | 0 | 1 |
| 0 | 1 | 0,18 | S | 0 | 0 | 1 | 1 | 1 | 1 | 1 | 1 | 0 | 1 | 0 |
| 0 | 1 | 0,18 | S | 0 | 0 | 1 | 1 | 1 | 1 | 1 | 1 | 1 | 1 | 0 |

### Interprétation des résultats

Dans ce modèle, l'haplotype majoritaire est celui dans lequel on détecte la présence des séquences SEQ ID NOs: 1 à 6, 9 et SEQ ID NO: 7 et l'absence de la séquence SEQ ID NO : 8. Cet haplotype est observé dans 186 génomes (185+1), soit 32,75% des 568 génomes de validation.

Par conséquent, la détermination de la présence et de l'absence desdites séquences dans le génome d'une souche *Mycobacterium tuberculosis* conduit à la bonne détermination (ou prédiction) du caractère « sensible » pour 185 génomes et avec 1 seule VME.

De la même manière, l'haplotype dans lequel on détecte la présence de l'ensemble des séquences SEQ ID NOs: 1 à 9 est observé dans 85 génomes, soit 14,96% des génomes de validation. La détermination de la présence et de l'absence desdites séquences permet ainsi de prédire le caractère « sensible » pour 82 génomes, et avec 3 VME.

### Performances du modèle

Au global, ce modèle permet de prédire sur la base de la présence ou de l'absence des séquences SEQ ID NO : 1 à 9, le caractère sensible ou résistant d'une souche *Mycobacterium tuberculosis* avec une spécificité de 87,22%, une sensibilité de 96,55% et avec un taux d'erreur à 5,63% (ME et VME).

### 2.6 Modèle rpoB-fabG1-gyrA- Rv1042c/Rv1149-embB-pncA-katG

Le tableau 6 ci-dessous présente les résultats de la susceptibilité par détection de la présence de mutations dans les gènes *rpoB, fabG1, gyrA, Rv1042c, Rv1149, embB, pncA* et *katG* dans la souche via la détection de la présence ou absence des séquences SEQ ID NOs: 1 à 12.

**Tableau 6**

| | | | | | | **SEQ ID NO** : | | | | | | | | | | | |
|---|---|---|---|---|---|---|---|---|---|---|---|---|---|---|---|---|---|
| | | | | | | **10** | **2** | **11** | **1** | **6** | **12** | **7** | **4** | **5** | **8** | **9** | **3** |
| **NbSR** | **NbSS** | **Prev** | **Pred** | **ME** | **VME** | **pncA (3.64)** | **rpoB (2.52)** | **pncA (2.61)** | **rpoB (2.04)** | **gyrA (1.46)** | **katG (1.23)** | **Rv1042c/Rv1149 (1.06)** | **fabG1 (0.91)** | **fabG1 (0.91)** | **Rv1042c/Rv1149 (0.84)** | **embB (0.71)** | **rpoB (0.43)** |
| 1 | 163 | 28,87 | S | 0 | 1 | 1 | 1 | 1 | 1 | 1 | 1 | 1 | 1 | 1 | 0 | 1 | 1 |
| 1 | 75 | 13,38 | S | 0 | 1 | 1 | 1 | 1 | 1 | 1 | 1 | 0 | 1 | 1 | 0 | 1 | 1 |
| 3 | 72 | 13,2 | S | 0 | 3 | 1 | 1 | 1 | 1 | 1 | 1 | 1 | 1 | 1 | 1 | 1 | 1 |
| 0 | 22 | 3,87 | S | 0 | 0 | 1 | 1 | 1 | 1 | 1 | 0 | 1 | 1 | 1 | 0 | 1 | 1 |
| 0 | 19 | 3,35 | S | 0 | 0 | 1 | 1 | 1 | 1 | 1 | 1 | 1 | 0 | 0 | 0 | 1 | 1 |
| 14 | 0 | 2,46 | R | 0 | 0 | 1 | 0 | 0 | 1 | 0 | 0 | 1 | 0 | 0 | 1 | 0 | 0 |
| 11 | 0 | 1,94 | R | 0 | 0 | 1 | 0 | 1 | 1 | 0 | 1 | 1 | 0 | 0 | 1 | 0 | 0 |
| 9 | 0 | 1,58 | R | 0 | 0 | 1 | 1 | 1 | 0 | 0 | 0 | 1 | 0 | 0 | 1 | 0 | 1 |
| 0 | 9 | 1,58 | S | 0 | 0 | 1 | 1 | 1 | 1 | 1 | 0 | 0 | 1 | 1 | 0 | 1 | 1 |
| 0 | 9 | 1,58 | S | 0 | 0 | 1 | 1 | 1 | 1 | 1 | 0 | 1 | 1 | 1 | 1 | 1 | 1 |
| 8 | 0 | 1,41 | R | 0 | 0 | 1 | 0 | 1 | 1 | 0 | 0 | 1 | 1 | 1 | 1 | 0 | 0 |
| 5 | 3 | 1,41 | R | 3 | 0 | 1 | 0 | 1 | 1 | 1 | 0 | 1 | 1 | 1 | 1 | 0 | 0 |
| 7 | 1 | 1,41 | R | 1 | 0 | 1 | 0 | 1 | 1 | 1 | 0 | 1 | 1 | 1 | 1 | 1 | 0 |
| 0 | 7 | 1,23 | S | 0 | 0 | 1 | 1 | 1 | 0 | 1 | 1 | 1 | 1 | 1 | 0 | 1 | 0 |
| 6 | 0 | 1,06 | R | 0 | 0 | 1 | 0 | 1 | 1 | 0 | 0 | 1 | 1 | 1 | 1 | 1 | 0 |
| 5 | 1 | 1,06 | R | 1 | 0 | 1 | 0 | 1 | 1 | 0 | 1 | 1 | 0 | 0 | 1 | 1 | 0 |
| 0 | 6 | 1,06 | S | 0 | 0 | 1 | 1 | 1 | 1 | 1 | 1 | 1 | 0 | 0 | 1 | 1 | 1 |
| 1 | 4 | 0,88 | S | 0 | 1 | 1 | 0 | 1 | 1 | 1 | 0 | 1 | 1 | 1 | 0 | 1 | 0 |
| 0 | 5 | 0,88 | S | 0 | 0 | 1 | 1 | 1 | 1 | 1 | 1 | 0 | 0 | 0 | 0 | 1 | 1 |
| 4 | 0 | 0,7 | R | 0 | 0 | 1 | 0 | 1 | 1 | 0 | 1 | 1 | 0 | 0 | 0 | 1 | 0 |
| 4 | 0 | 0,7 | R | 0 | 0 | 1 | 1 | 1 | 0 | 0 | 0 | 1 | 1 | 1 | 1 | 1 | 1 |
| 3 | 0 | 0,53 | R | 0 | 0 | 1 | 0 | 0 | 1 | 0 | 1 | 1 | 0 | 0 | 1 | 0 | 0 |
| 3 | 0 | 0,53 | R | 0 | 0 | 1 | 0 | 0 | 1 | 1 | 0 | 1 | 0 | 0 | 1 | 0 | 0 |
| 2 | 1 | 0,53 | R | 1 | 0 | 1 | 0 | 0 | 1 | 1 | 0 | 1 | 1 | 1 | 0 | 1 | 0 |
| 3 | 0 | 0,53 | R | 0 | 0 | 1 | 0 | 1 | 1 | 0 | 0 | 1 | 0 | 0 | 1 | 0 | 0 |
| 3 | 0 | 0,53 | R | 0 | 0 | 1 | 0 | 1 | 1 | 1 | 0 | 1 | 0 | 0 | 1 | 0 | 0 |
| 2 | 1 | 0,53 | R | 1 | 0 | 1 | 0 | 1 | 1 | 1 | 0 | 1 | 1 | 1 | 0 | 0 | 0 |
| 1 | 2 | 0,53 | S | 0 | 1 | 1 | 0 | 1 | 1 | 1 | 1 | 1 | 1 | 1 | 0 | 1 | 0 |
| 0 | 3 | 0,53 | R | 3 | 0 | 1 | 1 | 1 | 0 | 1 | 0 | 1 | 1 | 1 | 1 | 0 | 0 |
| 1 | 2 | 0,53 | S | 0 | 1 | 1 | 1 | 1 | 0 | 1 | 1 | 1 | 1 | 1 | 1 | 1 | 0 |
| 0 | 3 | 0,53 | S | 0 | 0 | 1 | 1 | 1 | 1 | 1 | 1 | 0 | 1 | 1 | 1 | 1 | 1 |
| 2 | 0 | 0,35 | R | 0 | 0 | 1 | 0 | 0 | 1 | 0 | 0 | 1 | 1 | 1 | 1 | 0 | 0 |
| 2 | 0 | 0,35 | R | 0 | 0 | 1 | 0 | 0 | 1 | 0 | 0 | 1 | 1 | 1 | 1 | 1 | 0 |
| 2 | 0 | 0,35 | R | 0 | 0 | 1 | 0 | 0 | 1 | 1 | 0 | 1 | 1 | 1 | 1 | 1 | 0 |
| 2 | 0 | 0,35 | R | 0 | 0 | 1 | 0 | 1 | 1 | 0 | 0 | 1 | 1 | 1 | 0 | 0 | 0 |
| 0 | 2 | 0,35 | S | 0 | 0 | 1 | 0 | 1 | 1 | 1 | 1 | 0 | 1 | 1 | 0 | 1 | 0 |
| 0 | 2 | 0,35 | S | 0 | 0 | 1 | 0 | 1 | 1 | 1 | 1 | 1 | 0 | 0 | 0 | 1 | 0 |
| 2 | 0 | 0,35 | R | 0 | 0 | 1 | 0 | 1 | 1 | 1 | 1 | 1 | 0 | 0 | 1 | 0 | 0 |
| 0 | 2 | 0,35 | S | 0 | 0 | 1 | 0 | 1 | 1 | 1 | 1 | 1 | 1 | 1 | 1 | 1 | 0 |
| 1 | 1 | 0,35 | S | 0 | 1 | 1 | 1 | 1 | 0 | 1 | 1 | 1 | 1 | 1 | 1 | 0 | 1 |
| 1 | 1 | 0,35 | S | 0 | 1 | 1 | 1 | 1 | 1 | 1 | 0 | 1 | 1 | 1 | 0 | 0 | 0 |
| 1 | 0 | 0,18 | R | 0 | 0 | 0 | 0 | 1 | 1 | 0 | 1 | 1 | 0 | 0 | 0 | 0 | 0 |
| 1 | 0 | 0,18 | R | 0 | 0 | 0 | 0 | 1 | 1 | 1 | 1 | 1 | 0 | 0 | 0 | 0 | 0 |
| 1 | 0 | 0,18 | R | 0 | 0 | 0 | 0 | 1 | 1 | 1 | 1 | 1 | 0 | 0 | 1 | 0 | 0 |
| 1 | 0 | 0,18 | R | 0 | 0 | 0 | 0 | 1 | 1 | 1 | 1 | 1 | 1 | 1 | 1 | 1 | 0 |
| 1 | 0 | 0,18 | R | 0 | 0 | 0 | 1 | 0 | 1 | 0 | 1 | 1 | 1 | 1 | 0 | 0 | 1 |
| 1 | 0 | 0,18 | R | 0 | 0 | 1 | 0 | 0 | 1 | 0 | 1 | 1 | 0 | 0 | 1 | 1 | 0 |
| 1 | 0 | 0,18 | R | 0 | 0 | 1 | 0 | 0 | 1 | 1 | 1 | 1 | 0 | 0 | 1 | 0 | 0 |
| 1 | 0 | 0,18 | R | 0 | 0 | 1 | 0 | 1 | 0 | 1 | 1 | 1 | 1 | 1 | 1 | 1 | 0 |
| 1 | 0 | 0,18 | R | 0 | 0 | 1 | 0 | 1 | 1 | 0 | 0 | 0 | 1 | 1 | 0 | 0 | 0 |
| 1 | 0 | 0,18 | R | 0 | 0 | 1 | 0 | 1 | 1 | 0 | 1 | 1 | 1 | 1 | 0 | 0 | 0 |
| 0 | 1 | 0,18 | S | 0 | 0 | 1 | 0 | 1 | 1 | 1 | 0 | 0 | 1 | 1 | 0 | 1 | 0 |
| 0 | 1 | 0,18 | R | 1 | 0 | 1 | 0 | 1 | 1 | 1 | 0 | 1 | 0 | 0 | 0 | 1 | 0 |
| 1 | 0 | 0,18 | S | 0 | 1 | 1 | 0 | 1 | 1 | 1 | 1 | 0 | 1 | 1 | 0 | 0 | 0 |
| 0 | 1 | 0,18 | S | 0 | 0 | 1 | 0 | 1 | 1 | 1 | 1 | 0 | 1 | 1 | 0 | 1 | 1 |
| 1 | 0 | 0,18 | S | 0 | 1 | 1 | 0 | 1 | 1 | 1 | 1 | 1 | 0 | 0 | 1 | 1 | 0 |
| 0 | 1 | 0,18 | S | 0 | 0 | 1 | 0 | 1 | 1 | 1 | 1 | 1 | 1 | 1 | 0 | 0 | 0 |
| 0 | 1 | 0,18 | S | 0 | 0 | 1 | 0 | 1 | 1 | 1 | 1 | 1 | 1 | 1 | 1 | 0 | 0 |
| 1 | 0 | 0,18 | R | 0 | 0 | 1 | 1 | 0 | 0 | 0 | 0 | 1 | 1 | 1 | 0 | 0 | 1 |
| 0 | 1 | 0,18 | S | 0 | 0 | 1 | 1 | 0 | 1 | 1 | 1 | 1 | 1 | 1 | 1 | 1 | 1 |
| 1 | 0 | 0,18 | R | 0 | 0 | 1 | 1 | 1 | 0 | 0 | 0 | 1 | 0 | 0 | 0 | 1 | 0 |
| 0 | 1 | 0,18 | R | 1 | 0 | 1 | 1 | 1 | 0 | 0 | 0 | 1 | 1 | 1 | 0 | 0 | 1 |
| 1 | 0 | 0,18 | R | 0 | 0 | 1 | 1 | 1 | 0 | 0 | 0 | 1 | 1 | 1 | 1 | 0 | 1 |
| 1 | 0 | 0,18 | R | 0 | 0 | 1 | 1 | 1 | 0 | 0 | 1 | 1 | 1 | 1 | 1 | 1 | 1 |
| 0 | 1 | 0,18 | S | 0 | 0 | 1 | 1 | 1 | 0 | 1 | 0 | 0 | 1 | 1 | 0 | 1 | 0 |
| 1 | 0 | 0,18 | R | 0 | 0 | 1 | 1 | 1 | 0 | 1 | 0 | 1 | 0 | 0 | 0 | 0 | 0 |
| 1 | 0 | 0,18 | R | 0 | 0 | 1 | 1 | 1 | 0 | 1 | 0 | 1 | 0 | 0 | 1 | 0 | 1 |
| 0 | 1 | 0,18 | R | 1 | 0 | 1 | 1 | 1 | 0 | 1 | 0 | 1 | 1 | 1 | 1 | 1 | 1 |
| 1 | 0 | 0,18 | S | 0 | 1 | 1 | 1 | 1 | 0 | 1 | 1 | 1 | 0 | 0 | 1 | 1 | 1 |
| 0 | 1 | 0,18 | S | 0 | 0 | 1 | 1 | 1 | 0 | 1 | 1 | 1 | 1 | 1 | 0 | 1 | 1 |
| 1 | 0 | 0,18 | S | 0 | 1 | 1 | 1 | 1 | 1 | 0 | 0 | 1 | 0 | 0 | 0 | 1 | 0 |
| 1 | 0 | 0,18 | R | 0 | 0 | 1 | 1 | 1 | 1 | 0 | 1 | 1 | 0 | 0 | 1 | 0 | 1 |
| 0 | 1 | 0,18 | S | 0 | 0 | 1 | 1 | 1 | 1 | 0 | 1 | 1 | 1 | 1 | 0 | 1 | 1 |
| 1 | 0 | 0,18 | S | 0 | 1 | 1 | 1 | 1 | 1 | 0 | 1 | 1 | 1 | 1 | 1 | 0 | 0 |
| 0 | 1 | 0,18 | S | 0 | 0 | 1 | 1 | 1 | 1 | 0 | 1 | 1 | 1 | 1 | 1 | 1 | 1 |
| 0 | 1 | 0,18 | S | 0 | 0 | 1 | 1 | 1 | 1 | 1 | 0 | 0 | 0 | 0 | 0 | 1 | 1 |
| 0 | 1 | 0,18 | S | 0 | 0 | 1 | 1 | 1 | 1 | 1 | 0 | 0 | 1 | 1 | 1 | 1 | 1 |
| 1 | 0 | 0,18 | S | 0 | 1 | 1 | 1 | 1 | 1 | 1 | 0 | 1 | 0 | 0 | 0 | 0 | 0 |
| 0 | 1 | 0,18 | S | 0 | 0 | 1 | 1 | 1 | 1 | 1 | 0 | 1 | 1 | 1 | 0 | 0 | 1 |
| 0 | 1 | 0,18 | S | 0 | 0 | 1 | 1 | 1 | 1 | 1 | 0 | 1 | 1 | 1 | 0 | 1 | 0 |
| 0 | 1 | 0,18 | S | 0 | 0 | 1 | 1 | 1 | 1 | 1 | 0 | 1 | 1 | 1 | 1 | 1 | 0 |
| 0 | 1 | 0,18 | S | 0 | 0 | 1 | 1 | 1 | 1 | 1 | 1 | 0 | 1 | 1 | 0 | 0 | 1 |
| 1 | 0 | 0,18 | S | 0 | 1 | 1 | 1 | 1 | 1 | 1 | 1 | 1 | 0 | 0 | 1 | 0 | 0 |
| 0 | 1 | 0,18 | S | 0 | 0 | 1 | 1 | 1 | 1 | 1 | 1 | 1 | 0 | 0 | 1 | 0 | 1 |

### Interprétation des résultats

Dans ce modèle, l'haplotype majoritaire est celui dans lequel on détecte la présence des séquences SEQ ID NOs : 1 à 6, 9 à 12 et SEQ ID NO : 7 et l'absence de la séquence SEQ ID NO : 8. Cet haplotype est observé dans 164 génomes (163+1), soit 28,87% des 568 génomes de validation.

Par conséquent, la détermination de la présence et de l'absence desdites séquences dans le génome d'une souche *Mycobacterium tuberculosis* conduit à la bonne détermination (ou prédiction) du caractère « sensible » pour 163 génomes et avec seulement 1 VME.

De la même manière, l'haplotype dans lequel on détecte la présence de l'ensemble des séquences SEQ ID NOs : 1 à 12 est observé dans 75 génomes, soit 13,2% des génomes de validation. La détermination de la présence et de l'absence desdites séquences permet ainsi de prédire le caractère « sensible » pour 72 génomes, et avec 3 VME.

### Performances du modèle

Au global, ce modèle permet de prédire sur la base de la présence ou de l'absence des séquences SEQ ID NOs : 1 à 12, le caractère sensible ou résistant d'une souche *Mycobacterium tuberculosis* avec une spécificité de 88,72%, une sensibilité de 97,01% et avec un taux d'erreur à 5,28% (ME et VME).

### Exemple 2 : Description des séquences utiles à la mise en oeuvre de la méthode selon l'invention

| **Nom** | **Séquence d'acides nucléiques** | **SEQ ID NO :** |
|---|---|---|
| rpoB unitig 1 | GACCAGAACAACCCGCTGTCGGGGTTGACCC | 1 |
| rpoB unitig 2 | GACAGACCGCCGGGCCCCAGCGCCGACAGTCG | 2 |
| rpoB unitig 3 | GACCCACAAGCGCCGACTGTCGGCGCTGGGGCCCG | 3 |
| FabG1 unitig 1 | CCGACAACCTATCGTCTCGCCGCGGCCGGGCC | 4 |
| FabG1 unitig 2 | GGCAGTCACCCCGACAACCTATCGTCTCGCCGCGGCCG GG | 5 |
| gyrA unitig | ACCACCCGCACGGCGACGCGTCGATCTACGA | 6 |
| Rv1042v/Rv11 49 unitig 1 | CGGCATCAACGGCTCGACCACGGCCCAGAACTCGTCCG AAATCACACCC | 7 |
| Rv1042v/Rv11 49 unitig 2 | GCGTCACCGGCCAATCCTCGCTGGCCAGTACC | 8 |
| embB unitig | CGGCGACTCGGGCCATGCCCAGGATGTAGCCGTCGTCC GACG | 9 |
| pncA unitig 1 | AACGCGGCGTCGATGAGGTCGATGTGGTCGGTA | 10 |
| pncA unitig 2 | CAGAAGTCGTTCTGCACGTCGACGATGATCA | 11 |
| katG unitig | GCACCGGAACCGGTAAGGACGCGATCACCAGCGGCATC GAGG | 12 |

## Revendications

1. Méthode de détermination de la susceptibilité d'une souche de l'espèce *Mycobacterium tuberculosis* au pyrazinamide, ledit méthode comprenant les étapes suivantes de :
- détermination de la présence dans le génome de ladite souche d'au moins une mutation dans le gène *rpoB* entre les positions 761112 et 761182,
- détermination du caractère sensible ou résistant de ladite souche en fonction de la présence ou non de ladite mutation.

2. Méthode selon la revendication 1, **caractérisée en ce qu'**elle comprend en outre la détermination de la présence dans le génome de ladite souche d'au moins une mutation dans le gène *fabG1* entre les positions 1673413 et 1673454.

3. Méthode selon la revendication 1 ou 2, **caractérisée en ce qu'**elle comprend en outre détermination de la présence dans le génome de ladite souche d'au moins une mutation dans le gène *gyrA* entre les positions 7552 et 7582.

4. Méthode selon l'une des revendications 1 à 3, **caractérisée en ce qu'**elle comprend en outre détermination de la présence dans le génome de ladite souche d'au moins une mutation dans le gène Rv1042c et sa région promotrice entre les positions 1165444 et 1165528 et/ou d'au moins une mutation le gène Rv1149 et sa région promotrice entre les positions 1277873 et 1277957.

5. Méthode selon la revendication 4, **caractérisée en ce qu'**elle comprend en outre une étape de détermination de la présence d'au moins une mutation dans le gène Rv1042c entre les positions 1165444 et 1165492 et d'au moins une mutation dans le gène Rv1149 entre les positions 1277909 et 1277957 et/ou une étape de détermination de la présence d'au moins une mutation dans le gène Rv1042c et sa région promotrice entre les positions 1165497 et 1165528 et d'au moins une mutation dans le gène Rv1149 et sa région promotrice entre les positions 1277873 et 1277904.

6. Méthode selon l'une des revendications 1 à 6, **caractérisée en ce qu'**elle comprend en outre les étapes suivantes de :
- détermination de la présence dans le génome de ladite souche d'au moins une mutation dans le gène *embB* entre les positions 4247581 et 4247622, et/ou
- détermination de la présence dans le génome de ladite souche d'au moins une mutation dans le gène *pncA* entre les positions 2288853 et 2289239, et/ou
- détermination de la présence dans le génome de ladite souche d'au moins une mutation dans le gène *katG* entre les positions 2155164 et 2155205.

7. Méthode selon l'une des revendications 1 à 6, **caractérisée en ce que** la détermination de la présence d'au moins une mutation dans le gène *rpoB* comprend la détermination de la présence d'au moins une séquence choisie parmi les séquences SEQ ID NO :1, SEQ ID NO :2 et SEQ ID NO :3.

8. Méthode selon l'une des revendications 2 à 7, **caractérisée en ce que** la détermination de la présence d'au moins une mutation dans le gène *fabG1* comprend la détermination de la présence d'au moins une séquence choisie parmi les séquences SEQ ID NO : 4 et SEQ ID NO :5.

9. Méthode selon l'une des revendications 3 à 8, **caractérisée en ce que** la détermination de la présence d'au moins une mutation dans le gène *gyrA* comprend la détermination de la présence de la séquence SEQ ID NO :6.

10. Méthode selon l'une des revendications 4 à 9, **caractérisée en ce que** la détermination de la présence d'au moins une mutation dans région génomique comprise entre les positions 1165444 et 1165528 et/ou d'au moins une mutation dans la région génomique entre les positions 1277873 et 1277957 comprend la détermination de la présence d'au moins une séquence choisie parmi les séquences SEQ ID NO : 7 et SEQ ID NO :8.

11. Méthode selon l'une des revendications 6 à 10, **caractérisée en ce que** la détermination de la présence d'au moins une mutation dans le gène *embB* comprend la détermination de la présence de la séquence SEQ ID NO :9.

12. Méthode selon l'une des revendications 6 à 11, **caractérisée en ce que** :
- la détermination de la présence d'au moins une mutation dans le gène *pncA* consiste à déterminer la présence d'au moins une des séquences SEQ ID NO :10 et SEQ ID NO :11,
- la détermination de la présence d'au moins une mutation dans le gène *katG* consiste à déterminer la présence de la séquence SEQ ID NO :12.

13. Méthode selon la revendication 12, **caractérisée en ce qu'**il comprend une étape de détermination de la présence des séquences SEQ ID NOs : 1 à 12 dans les gènes respectifs et de détermination du caractère sensible ou résistant de ladite souche en fonction de la présence ou non desdites séquences.

14. Méthode selon l'une quelconque des revendications 1 à 13, **caractérisée en ce que** les étapes de détermination de la présence des mutations sont réalisées par rapport au génome sauvage de référence de *Mycobacterium tuberculosis* H37Rv (référence NC_000962.3).

15. Méthode selon l'une quelconque des revendications 1 à 14, dans lequel l'étape de détermination de la présence d'au moins une mutation dans lesdits gènes est réalisée par une méthode d'hybridation, d'amplification ou de séquençage.

16. Méthode selon l'une quelconque des revendications 1 à 15, **caractérisée en ce qu'**elle est mise en oeuvre par un ordinateur.

17. Appareil de traitement de donnée comprenant :
- des moyens pour mettre en oeuvre la méthode selon la revendication 16, notamment des moyens pour déterminer la présence de mutations dans le gène *rpoB* de la souche *Mycobacterium tuberculosis* entre les positions 761112 et 761182, et/ou des moyens permettant de comparer le séquence dudit gène avec le génome de référence *Mycobacterium tuberculosis* H37Rv (référence NC_000962.3), et/ou des moyens pour identifier la présence des séquences SEQ ID NOs 1 à 3 dans le génome de la souche *Mycobacterium tuberculosis,* et/ou des moyens pour fournir une donnée de sortie de la présence ou de l'absence desdites séquences, lorsque lesdits moyens sont mis en oeuvre ou pilotés par ordinateur, ou
- un processeur adapté à ou configuré pour exécuter la méthode mise en oeuvre par ordinateur selon la revendication 16, en particulier un processeur adapté à ou configuré pour exécuter les étapes de ladite méthode.

18. Kit comprenant des moyens de détection d'au moins une séquence choisie parmi les séquences SEQ ID NO :1 à 3, et éventuellement au moins une autre séquence choisie parmi les séquences SEQ ID NOs : 4 à 12, lesdits moyen étant de préférence des amorces et/ou des sondes.

19. Utilisation du kit selon la revendication 18 pour la détermination de la susceptibilité d'une souche *Mycobacterium tuberculosis* au pyrazinamide.

20. Utilisation des séquences SEQ ID NOs 1 à 3, et de préférence également des séquences SEQ ID NOs 4 à 12, pour la détermination de la susceptibilité d'une souche *Mycobacterium tuberculosis* au pyrazinamide.
